# EUROPEAN PATENT APPLICATION

(11) **EP 4 574 027 A2**
(43) Date of publication of application: **25.06.2025**
(21) Application number: 25172298.9
(22) Date of filing: 13.05.2020
(51) Int. Cl.: A61B 5/0215

(54) **PRESSURE SENSING GUIDEWIRES, SYSTEMS AND METHODS FOR STRUCTURAL HEART PROCEDURES**

(30) Priority: 17.05.2019 US 201962849768 P; 28.10.2019 US 201962926737 P
(62) Divisional of application: 20809315.3
(71) Applicant: Opsens Inc., Quebec City QC G1P 4S3 (CA)
(72) Inventor: LALANCETTE, Sebastien, Quebec City, G1P 4S3 (CA); DELAND, Maxime, Picard, Quebec City, G1P 4S3 (CA); BELLEVILLE, Claude, Quebec City, G1P 4S3 (CA)
(74) Representative: Boult Wade Tennant LLP

(57) **Abstract**

There is described a pressure guidewire comprising an outer tube comprising a sensor housing and a coil portion; a connector tube positioned radially inward of the outer tube, the connector tube comprising a tube wall and a lumen extending therethrough; a core wire positioned radially inward of the outer tube and longitudinally spaced apart from a distal end of the connector tube, the core wire comprising a reduced diameter portion; a pressure sensor assembly comprising a pressure sensor and a pressure wire lead extending from the pressure sensor toward a proximal end of the pressure guidewire, the pressure sensor being distal of the distal end of the connector tube, the pressure wire lead at least partially extending through the lumen of the connector tube; and a distal tip at a distal end of the outer tube.

## Description

### INCORPORATION BY REFERENCE TO ANY PRIORITY APPLICATIONS

This application claims the benefit of U.S. Provisional Application No. 62/849,768, filed May 17, 2019, and U.S. Provisional Application No. 62/926,737, filed October 28, 2019, all of which are incorporated by reference in their entirety herein.

Any and all applications for which a foreign or domestic priority claim is identified in the Application Data Sheet as filed with the present application are hereby incorporated by reference under 37 C.F.R. § 1.57.

### BACKGROUND

### Field

This application is directed to a structural heart guidewire that is configured to sense blood pressure to provide information about blood flow through a heart valve before, during and/or immediately after a structural heart procedure.

### Description of the Related Art

Guidewires are known for delivering catheters to many vascular locations in the body. Access to vascular locations is facilitated by a combination of mechanical properties such as flexibility, pushability and torqueability. It is known for coronary procedures to include a pressure sensor to enable a measure of blood flow through a static occlusion to help a cardiologist determine whether to treat a patient.

While pressure sensing around static lesions in coronary vessels is known such concepts have not been applied to structural heart procedures, such as for treatment of heart valves and improving heart pumping function. Pumping function has been addressed with mechanical pumps of various sorts. Heart valves have historically been treated by open heart surgery. Presently, however, heart valves are more and more replaced by cardiologists using catheters upon which percutaneous heart valves are mounted and by which such valves are delivered.

### SUMMARY

While pressure measuring coronary guidewires have been described and marketed for many years, structural heart guidewires have not been developed. Accordingly, structural heart guidewires are needed for enabling a cardiologist to improve structural heart procedures.

During structural heart procedures, a downstream pressure curve and an upstream pressure curve can be used to determine a condition of a heart valve, a status of blood flow through a heart valve and in some cases to determine how and when to treat a patient. Depending on the valve to be treated and the approach, in some implementations, the downstream pressure curve can be provided by a guide catheter pressure sensor, a pressure guidewire or another device capable of sensing pressure. The upstream pressure curve can be provided by a pressure guidewire or other device capable of sensing pressure upstream to the downstream pressure measurement. In other implementations, the upstream pressure curve can be provided by a guide catheter pressure sensor, a pressure guidewire or another device capable of sensing pressure. The downstream pressure curve can be provided by a pressure guidewire or other device capable of sensing pressure downstream to the upstream pressure measurement.

For example, some methods for evaluating a heart valve include accessing a blood flow passage of a patient at an access point. The access point may be a femoral artery, radial artery, femoral vein, radial vein, left ventricle apex, or otherwise. A pressure guidewire may be advanced through the access point to a location adjacent to a treatment site of the patient, for example the heart valve to be assessed, treated, or replaced. A pressure sensing device separate from the pressure guidewire may be advanced to the opposite side of the treatment site, e.g., to the side of a heart valve opposite to the side of the valve where a pressure sensing device located toward a distal tip of the pressure guidewire is located. The pressure sensing device may comprise or may be disposed in an aortic pigtail catheter, a guide catheter, a pressure guidewire, or another device capable of sensing pressure. Treatment devices, such as a balloon or replacement heart valve, may be advanced over the pressure guidewire. In some implementations, the pressure sensing device may sense pressure on a first side of the heart valve, e.g. in the aorta or atrium, and the pressure guidewire may sense pressure on a second side of the heart valve, e.g. in the left ventricle or the right ventricle. In some implementations, the pressure sensing device may sense pressure in a heart chamber and the pressure guidewire may sense pressure in a blood flow passage on an opposite of a heart valve, e.g., in a second heart chamber or in the aorta. A specific example includes positioning the pressure sensing device in the left ventricle to sense pressure therein and positioning the pressure guidewire in the aorta to sense pressure therein to evaluate the aortic valve from a transapical heart access approach. Another specific example includes positioning the pressure sensing device in the left ventricle to sense pressure therein and positioning the pressure guidewire in the left atrium to sense pressure therein to evaluate the mitral valve from a transapical heart access approach. The pressure measurements may be used to measure a valve state condition, such as pressure gradient across the heart valve and/or valve regurgitation.

The methods described herein may include equalizing pressure measurements between the pressure sensing device and the pressure guidewire. Pressure equalization may take place in any location such as the aorta or the left ventricle. Equalizing pressure measurements may include automatically or manually adjusting a phase delay between the pressure curves generated from the pressure sensing device and the pressure guidewire.

Some methods described herein are directed towards assessing and/or treating a cardiac and/or a cardiovascular condition. In some cases, the method involves treating a structural heart condition. For example, the method may include: accessing a blood flow passage of a patient at an access point, advancing an access catheter through the access point to a location in the heart, advancing a pressure guidewire through the access catheter, and/or sensing pressure using the pressure guidewire. The method may also include inducing rapid pacing through the pressure guidewire. For example, current may be delivered from a proximal segment of the pressure guidewire and through a core wire of the pressure guidewire to a distal segment of the pressure guidewire. The access catheter or other delivery catheter may insulate the patient from current in the rapid pacing pressure guidewire. In some configurations, the pressure guidewire may include an insulator along at least a portion of the pressure guidewire, for example a polymeric layer such as a PTFE layer can insulate the patient from the rapid pacing pressure guidewire where the current application is not desired. By combining pressure sensing with rapid pacing capabilities, these methods eliminate the need for a separate pacing device and/or for exchange of such devices to sequentially provide these capabilities.

Various pressure guidewire configurations are suitable for the pressure sensing methods described herein. These pressure guidewires may guide other catheters advanced over the pressure guidewires. A distal segment of the catheter may include a curvature to provide an atraumatic tip. The pressure guidewire may include a distal tip to enclose a distal end of the pressure guidewire, e.g., to prevent fluid flow or passage of structures through the distal end of the pressure guidewire.

For example, a guidewire may include a connector tube and a core wire extending distally of a distal end of the connector tube. The guidewire may include a sensor assembly having a sensor (e.g., a pressure sensor) and a sensor housing positioned over the sensor. An insulator portion may surround at least a portion of the connector tube and extend proximally of the sensor housing. The connector tube and/or the core wire may have a uniform diameter or a reduced diameter portion, for example a tapered portion. Optionally, the guidewire may include a distal tip at a distal end of the guidewire.

In some configurations, a coil portion may at least partially surround the core wire. The insulator portion and the coil portion may have the same outer diameter.

In some configurations, at least one conductive section (e.g., one, two, three, or more) of the connector tube may be exposed from the insulator portion. At least one conductive section may be located outside of the patient during the procedure. The at least one conductive section may include spaced apart first and second conductive sections. For example, the first conductive section may be located at a proximal end of the guidewire and the second conductive section may be located distal of the first conductive section.

Some of the pressure guidewires described herein may include an outer tube having a lumen extending through the outer tube. At least a portion of the outer tube includes a coil portion and/or connector tube. The pressure guide wire may also include a core wire extending through at least a portion of the lumen of the outer tube. In some configurations, the core wire may extend substantially the entire length or the entire length of the lumen of the outer tube. The core wire may include a reduced diameter portion, such as a tapered portion. The pressure guide wire may also include a pressure sensor assembly having a pressure sensor and one or more pressure wires leads extending from the pressure sensor toward a proximal end of the pressure guidewire. For example, the pressure sensor may be an optical sensor, electrical, MEMS, or a membrane-based sensor, and the pressure wire lead(s) may be an optical fiber or an electrical wire. The pressure sensor may be positioned radially between the reduced diameter portion of the core wire and the coil portion of the outer tube. The pressure sensor may be disposed within a sensor housing or the outer tube itself may provide a sensor housing. The pressure sensor may be exposed to or in pressure communication with blood flow outside the pressure guidewire through the spacing in the coil portion and/or through one or more openings in the sensor housing.

At least a portion of at least one pressure wire lead may not be concentric with the outer tube. For example, a first section of the pressure wire lead may be concentric with the outer tube and a second section of the pressure wire lead may be off-axis relative to a longitudinal axis of the outer tube. The second section may be positioned radially outward of the core wire. For example, in the distal region of the pressure guidewire where the core wire has a reduced diameter, there may be space between the core wire and the outer tube for the pressure sensor to be positioned off-axis relative to the longitudinal axis of the outer tube. When the pressure sensor is located in the distal region of the pressure guidewire, the pressure guidewire is capable of measuring pressure at a position more centrally located in the chamber of the heart while the core wire maintains structural integrity in the distal region. However, it may be beneficial for at least a portion of the pressure wire lead to be concentric with the outer tube to facilitate connection to an optical or other connector at a proximal end of the pressure guidewire.

The outer tube may include an opening configured to permit at least one pressure wire lead to transition from the first section that is concentric with the outer tube to the second section that is not concentric with the outer tube. The opening may be a partial thickness cut out or extend through the full thickness of the outer tube. If the opening extends through the full thickness of the outer tube, the opening may be sealed, e.g. using adhesive, to prevent fluid from flowing into the pressure guidewire through the opening.

In some implementations, current may be delivered through the core wire to a conductive surface on an outside of the guidewire to induce rapid pacing. When the core wire extends the substantially entire or entire working length of the pressure guidewire, the current generator may deliver current directly to the core wire or to an exposed conductor in contact directly or indirectly with a proximal portion of the core wire. Additionally or alternatively, the current may be delivered to a conductive tube and/or coil and then directly or indirectly transferred to the core wire, for example through a separate conductive connector. In some configurations, the outer tube of the pressure guidewire may include an insulator along at least a portion of the pressure guidewire, for example a polymer layer such as PTFE, to insulate the patient from the core wire.

Some of the pressure guidewires described herein include connector tube, a core wire, a coil portion, and/or a pressure sensor assembly. The connector tube may extend from a proximal end of the pressure guidewire such that a current generator may be connected to the connector tube. The core wire may extend distally of a distal end of the connector tube, for example through the distal end of the connector tube or distal of the distal end of the connector tube. The core wire may include a reduced diameter portion such as a tapered portion. In some implementations, current may be directly or indirectly delivered from the connector tube to the core wire for rapid pacing. For example, current may be delivered from the connector tube to the core wire via a separate connector from the connector for the optical connection when using optical sensing.

The coil portion may be positioned distal to the distal end of the connector tube and surround at least a portion of the core wire. The coil portion may include a sensor housing section, e.g. a tube or weld, that is stiffer than another section or remainder of the coil portion. The pressure sensor of the pressure sensor assembly may be disposed within the sensor housing section of the coil portion. In this configuration, the sensor housing section of the coil portion may include one or more openings to allow the blood or another fluid in pressure communication with the blood to reach the pressure sensor.

The pressure sensor assembly may include a pressure sensor and one or more pressure wires leads extending from the pressure sensor toward the proximal end of the pressure guidewire. For example, the pressure sensor may be an optical sensor, electrical, MEMS, or a membrane-based sensor. The pressure sensor may be positioned radially between the reduced diameter portion of a core wire and a coil portion such that fluid may flow through a space in the coil portion to the pressure sensor. In some configurations, the pressure sensor assembly may include a separate pressure housing disposed over the pressure sensor.

The pressure wire(s) lead(s) may be an optical fiber or an electrical wire. A first section of at least one pressure wire lead may be concentric with the connector tube and a second section of the pressure wire lead may be off-axis relative to a longitudinal axis of the connector tube. The second section of the pressure wire lead may be positioned radially outward of the core wire. The tube wall of the connector tube may include an opening to permit the pressure wire lead to transition from the first section that is concentric with the connector tube to the second section that is off-axis relative to the longitudinal axis of the connector tube. The opening may be a partial thickness cut out or extend through the full thickness of the connector tube. If the opening extends through the full thickness of the outer tube, the opening may be sealed to prevent fluid from flowing into the pressure guidewire through the opening. In other configurations, the pressure guidewire may include a separate connector with an opening to permit the pressure wire lead to transition from the first section that is concentric with the connector tube to the second section that is off-axis relative to the longitudinal axis of the connector tube.

Some of the pressure guidewire discussed herein include an outer tube, connector tube positioned radially inward of the outer tube, a pressure sensor assembly, and/or a distal tip at the distal end of the outer tube. The outer tube may have a uniform or substantially uniform diameter. A core wire may be positioned distal to the connector tube. The core wire may have a reduced diameter portion such as a tapered portion. The pressure sensor assembly may include a pressure sensor positioned distal of the connector tube, for example radially between a coil portion of the outer tube and the core wire. The pressure sensor assembly may also include one or more pressure wires leads extending from the pressure sensor and through the connector tube lumen.

The pressure guidewire may also include a sensor housing, for example in the outer tube or over the pressure sensor but within the outer tube. The sensor housing may include at least one opening to allow blood or other fluid to flow to the pressure sensor. In this configuration, the pressure guidewire may include a second coil portion extending proximally from the sensor housing toward a proximal end of the pressure guidewire. The coil portions of the outer tube may extend along a majority of a working length of the pressure guidewire or substantially the entire working length of the pressure guidewire. A proximal end of the connector tube may be exposed from a proximal end of the second coil portion to facilitate rapid pacing. For example, less than ten percent, or less than five percent, of a length of the connector tube may be exposed from the proximal end of the second coil portion.

### BRIEF DESCRIPTION OF THE DRAWINGS

These and other features, aspects and advantages are described below with reference to the drawings, which are intended for illustrative purposes and should in no way be interpreted as limiting the scope of the embodiments. Furthermore, various features of different disclosed embodiments can be combined to form additional embodiments, which are part of this disclosure. In the drawings, like reference characters denote corresponding features consistently throughout similar embodiments. The following is a brief description of each of the drawings.
Figures 1A-1F are schematic diagrams of a pressure guidewire deployed in a heart;
Figure 2A is a schematic view of a system including a console and a guidewire adapted for facilitating delivery of a structural heart device;
Figure 2B is a plan view of a coiled distal tip of a pressure sensing guidewire that can be incorporated into the system of Figure 2;
Figure 2C is a transverse cross-sectional view a system including a system including an aortic pigtail catheter and a guide catheter for a TAVR delivery system;
Figure 2D is a transverse cross-sectional view a system including a system including a guide catheter for a TMVR delivery system;
Figures 2E-2F are a schematic views of a pressure sensing guidewire in a method wherein the guidewire being used for rapid pacing.
Figure 3 is a schematic view of one of the variations of the pressure sensing guidewire shown in Figure 2B;
Figure 4 is a cross-sectional view of another one of the variations of the pressure sensing guidewire shown in Figure 2B;
Figure 5 is a schematic view of another one of the variations of the pressure sensing guidewire shown in Figure 2B;
Figure 6 is a cross-sectional view of another one of the variations of the pressure sensing guidewire shown in Figure 2B;
Figure 7 is a schematic view of another one of the variations of the pressure sensing guidewire shown in Figure 2B;
Figure 8 is a schematic view of another one of the variations of the pressure sensing guidewire shown in Figure 2B; and
Figure 9 is a cross-sectional view of another one of the variations of the pressure sensing guidewire shown in Figure 2B.
Figure 10A is a cross-sectional view of another one of the variations of the pressure sensing guidewire shown in Figure 2B.
Figure 10B is a cross-sectional view of another one of the variations of the pressure sensing guidewire shown in Figure 2B.
Figure 10C is a cross-sectional view of another one of the variations of the pressure sensing guidewire shown in Figure 2B.

### DETAILED DESCRIPTION

This application is directed to systems and methods for providing pressure curves during surgical heart procedures, including valvuloplasty procedures, transcatheter aortic valve replacement (TAVR) procedures sometimes also called transcatheter aortic valve implantation (TAVI) procedures, and transcatheter mitral valve replacement (TAMR) procedures. The systems and methods can be used to aid a cardiologist in completing critical aspects of a structural heart procedure. The embodiments herein can be used to convey by a user interface output, e.g., graphically, the condition of a heart valve before, during and/or immediately after the deployment of structural heart device such as an aortic valve, a mitral valve or another heart valve. The embodiments herein can be used to convey the nature of blood flow through a heart valve before, during and/or immediately after the deployment of structural heart device such as an aortic valve, mitral valve or another heart valve. Novel displays provide an intuitive and/or immediate sense of a condition of the patient to simplify and to expedite procedures and to increase the success thereof.

The pressure measurements obtained from the systems and methods described herein may be used to calculate a heart valve or blood flow index, such as a valve regurgitation index or a pressure gradient across a natural heart valve, a previous placed replacement heart valve or a replacement heart valve being currently implanted. The valve regurgitation index and pressure gradient enable the cardiologist to properly evaluate the heart valve. During systole, a higher pressure gradient across the aortic valve (or lower pressure in the aorta) may be indicative of greater valve calcification. A lower regurgitation index at the end of diastole may be indicative of greater regurgitation.

### I. EXAMPLE METHODOLOGIES

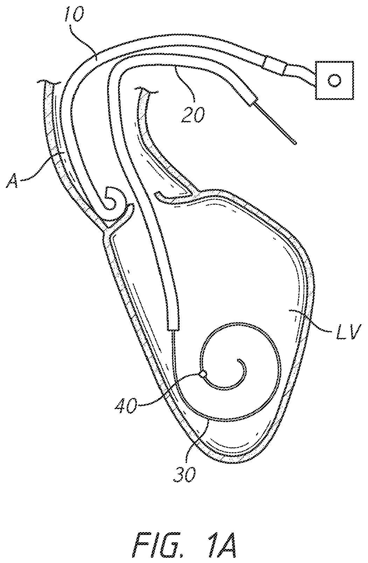

Figures 1A-1F illustrate various methods of accessing a heart during a structural heart procedure. One of the pressure guidewire 30 or the pressure sensing device (e.g., a pigtail catheter 10 or access catheter 20) may be used to calculate an upstream pressure curve (with respect to flow) and the other of the pressure guidewire 30 or the pressure sensing device may be used to calculate a downstream pressure curve (with respect to flow). Although certain methods are described below with respect to particular heart valves and approaches to access, similar systems may be used to evaluate other valves such as the tricuspid valve or the pulmonary valve.

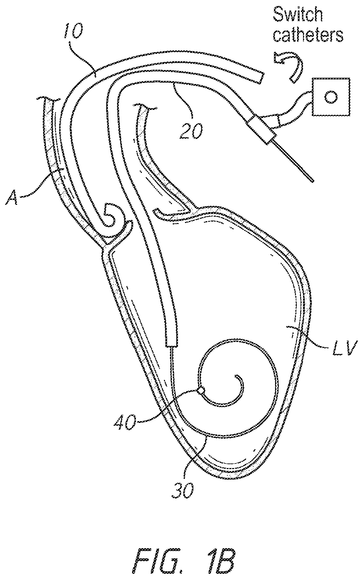

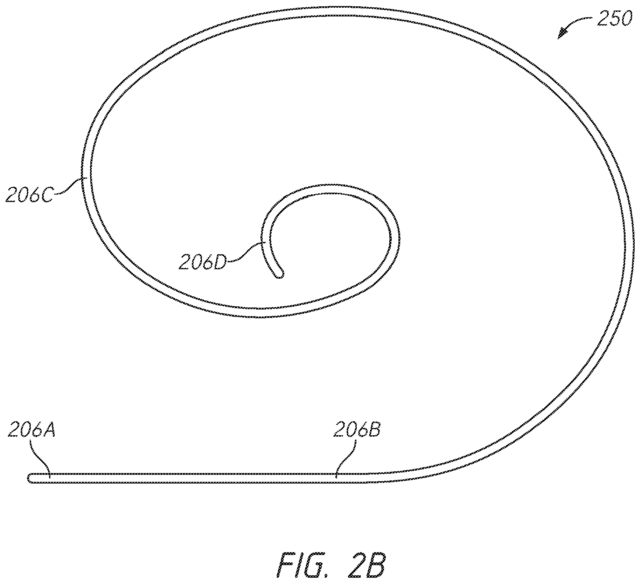

Figure 1A illustrates a system and method for measuring the performance of an existing or a replacement aortic heart valve. An existing heart valve can be a natural but diseased valve or a previously implanted replacement heart valve that is being assessed in a subsequent procedure. As shown, a pigtail catheter 10 may be positioned downstream of a treatment site, for example downstream of an aortic valve in an aorta A, to provide the downstream pressure curve. The pigtail catheter 10 may also be used to deliver contrast media to facilitate visualization of the treatment site. An access catheter 20 may be delivered to the heart from the same or different access site as the pigtail catheter 10. The access catheter 20 or a separate delivery catheter exchanged with the access catheter 20 may be used to advance a valve dilation balloon, replacement valve, and or other device to the treatment site. A pressure guidewire 30 may extend through the access catheter 20 to a position upstream of the treatment site, for example in left ventricle LV, to provide the upstream pressure curve. The pressure guidewire 30 may include a pressure sensor 40 anywhere along a distal segment of the pressure guidewire 30, for example within an atraumatic curvature, at the transition to the atraumatic curvature, or proximal of the atraumatic curvature (see Figure 2B). Prior to entering the heart, access is provided using an arterial approach, such as a femoral or a radial approach. Figure 1B illustrates a similar configuration to Figure 1A except one or both of the pigtail catheter 10 and/or the access catheter 20 can be used to provide pressure reading with the use of the external pressure sensing. Catheter 20 can allow the measurement of the downstream pressure, similar to the pressure read by the pressure guidewire 30. This configuration can be used to equalize the external pressure sensor with the pressure guidewire. Alternatively, any other delivery catheter exchanged with the access catheter may be used to provide the downstream pressure curve. In some cases, the downstream pressure output can be received by a console that can be coupled with the pressure signal of either or both of the pigtail catheter 10 and the access catheter 20.

It can be important to equalize the pressure readings between the downstream and the upstream pressure sensing devices. Equalization may be done in terms of pressure accuracy (gain and offset), but also in terms of phase delay between the two pressure curves. For example, pressure readings may be taken from the downstream and upstream pressure sensing devices in the same general anatomical region and the pressure measurements may be manually or automatically adjusted for the phase delay between the two pressure curves. As shown in Figure 1B, the pressure measurements for equalization may be taken from the left ventricle LV. In this approach, the downstream pressure output is provided by the access catheter 20 and the upstream pressure output is provided by the pressure guidewire 30. The sensing feature of the access catheter 20 (e.g., a distal end of a column of fluid in the catheter 20) is advanced to be adjacent to the sensing feature of the pressure guidewire 30. The sensing features of the access catheter 20 and the pressure guidewire 30 can be confirmed to be placed in the left ventricle LV. The sensing features of the access catheter 20 and the pressure guidewire 30 can be confirmed to be in a similar position in the left ventricle LV.

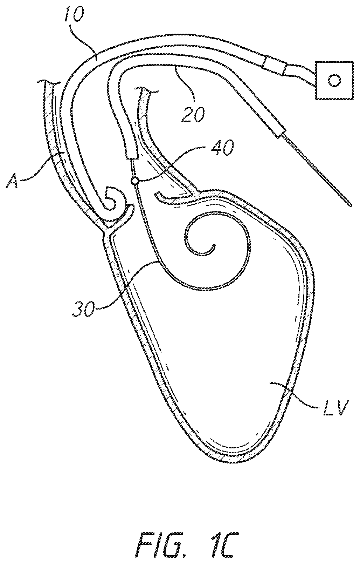

Figure 1C illustrates a similar configuration to Figure 1A except the pressure sensor 40 is located proximal of the atraumatic curvature of the pressure guidewire 30. For example, the sensing feature of the pigtail catheter 10 (e.g., a distal end of a column of fluid in the catheter 10) is advanced to be adjacent to the sensing feature of the pressure guidewire 30. The sensing features of the pigtail catheter 10 and the pressure guidewire 30 can be confirmed to be placed in the aorta A. In this configuration, pressure equalization may be performed in the aorta A. After pressure equalization, the pressure guidewire 30 may be advanced into the left ventricle LV to provide the upstream pressure curve while the pigtail catheter 10 remains in the aorta A to provide the downstream pressure curve.

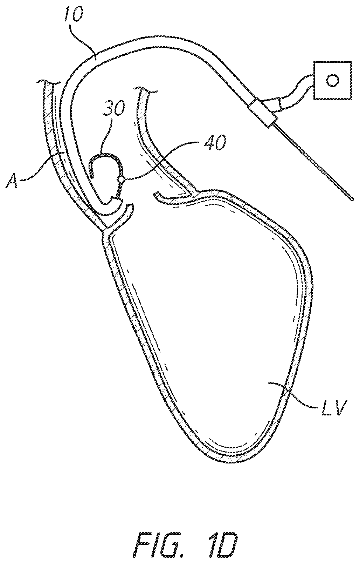

In Figure 1D, pigtail catheter 10 may be positioned in the aorta A to provide the downstream pressure curve. The pressure guidewire 30 extends through the pigtail catheter 10 in this embodiment to provide the upstream pressure curve. In this configuration, pressure equalization may be performed in the aorta A. For example, the sensing feature of the pressure guidewire 30 can be advanced to the end of a fluid column in the pigtail catheter 10 or just distal thereto. The signals from the sensing feature of the pressure guidewire 30 and the fluid column can be compared to equalize them. After pressure equalization, the pressure guidewire 30 may be retrieve from the aortic pigtail and insert in the left ventricle via the access catheter like it is usually done while the pigtail catheter 10 remains in the aorta A to provide the downstream pressure curve.

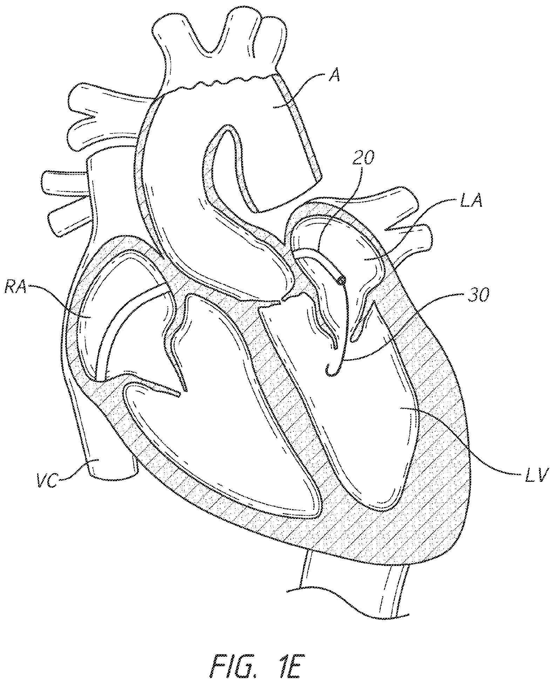

The systems described herein may also be used to measure the performance of an existing or replacement mitral valve. For example, as shown in Figure 1E, the access catheter 20 may be advanced through the venous vasculature, e.g., through an inferior or superior vena cava VC, e.g., from a femoral approach, to a right atrium RA. The access catheter 20 may then be advanced through an atrial septum to a position in a left atrium LA. In some variations, the access catheter 20 may be configured to provide access through a patent foramen ovale or may be configured to track a guidewire or device that has provided such access. The access catheter 20 or a separate delivery catheter exchanged with the access catheter 20 may be used to advance a valve dilation balloon, replacement valve, and or other device to the treatment site. The pressure guidewire 30 may extend through the access catheter 20 to the left ventricle LV. The access catheter 20 may provide pressure signals that can be used to generate an upstream pressure curve, while the pressure guidewire 30 provides pressure signals that can be used to generate a downstream pressure curve. Alternatively, any other delivery catheter exchanged with the access catheter may be used to provide the upstream pressure curve.

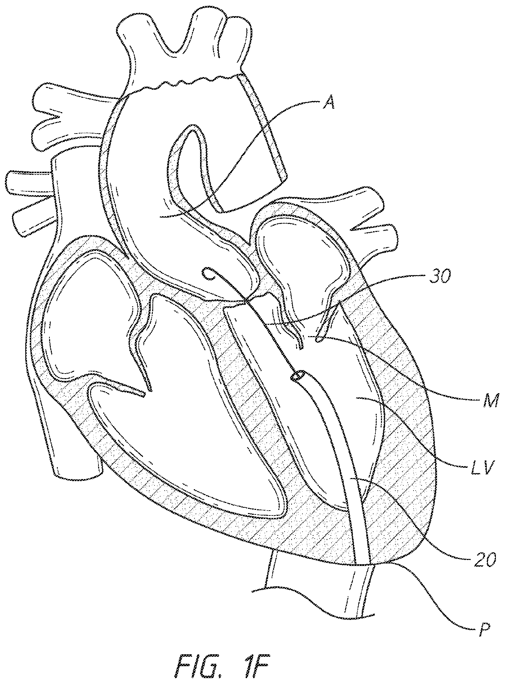

Similar systems may be used in an apical approach for aortic or mitral valve procedures. For example, as shown in Figure 1F, the access catheter 20 may access the left ventricle LV through the apex P of a heart. A separate device (not shown) can be used to open a pathway through the apex P. The access catheter 20 can be advanced through such as device. The access catheter 20 or a separate delivery catheter exchanged with the access catheter 20 may be used to advance a valve dilation balloon, replacement valve, and or other device to the treatment site. The pressure guidewire 30 may extend through the access catheter 20 to the aorta A in an aortic valve procedure. The access catheter 20 may provide the pressure signals that can be used to calculate an upstream pressure curve, while the pressure guidewire 30 can provide signals that can be used to calculate a downstream pressure curve. Alternatively, any other delivery catheter exchanged with the access catheter may be used to provide the upstream pressure curve.

Although FIG. 1F shows an aortic valve assessment or procedure via the apex P of the heart, the pressure guidewire 30 can be advanced through the mitral valve M such that the sensing feature thereof is in the left atrium. In this way, the pressure guidewire can provide pressure signals that can be used to calculate a left atrial pressure curve (proximal or upstream pressure curve from the perspective of flow). The access catheter 20 can generate pressure signals that can be used to calculate a left ventricle pressure curve (distal or downstream pressure curve from the perspective of flow).

During valve dilation procedures, sometimes called a valvuloplasty, or a valve implantation procedure, natural circulation through the heart valve may be blocked by the valvuloplasty balloon, valve replacement delivery system, or other treatment device. However, when the heart is pumping, pressure from the left ventricle LV or compression of the heart muscle may drive the treatment device back into the aorta A making it difficult to properly position the treatment device. Rapid pacing or defibrillating the left ventricle LV can reduce the pressure gradient between the aorta A and the left ventricle LV and also heart muscle forces and allow the clinician to complete the procedure. Conventional rapid pacing may involve introducing a temporary pace maker to the heart, but this usually requires a separate access point, for example a venous access point. Temporary pace makers may also burn the heart causing other complications. Instead, the pressure guidewire 30 may be used to perform the rapid pacing. As explained above, the pressure guidewire 30 may be introduced through the same access point as the access catheter 20 or other delivery catheter, which reduces the total number of access points. A current may be delivered to a proximal segment pressure guidewire and transmitted to a distal segment of the pressure guidewire via connector tube and/or the core wire, as explained in further detail below. The access catheter 20 or other delivery catheter may insulate at least an intermediate segment of the rapid pacing pressure guidewire 30 from the patient to prevent burns. Alternatively or additionally, the pressure guidewire 30 may include an insulator portion to isolate the pressure guidewire 30. As shown in Figure 2B, the distal segment of the pressure guidewire may include a curvature allowing the current to contact ventricle walls in multiple locations.

### II. OVERVIEW OF PRESSURE WIRE SYSTEMS AND THEIR USE

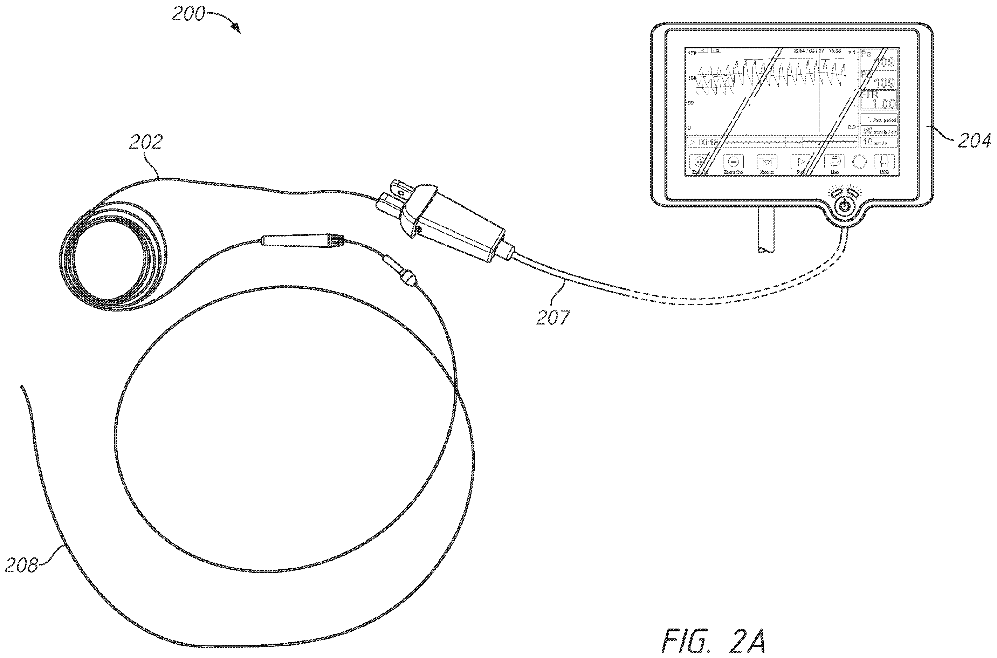

Figure 2A illustrates a diagnostic system 200 that can be used in the vasculature of a patient. The diagnostic system 200 is configured to determine whether the extent of valve damage is great enough to indicate that a balloon dilation (e.g., valvuloplasty), valve replacement or other catheter intervention ought to be performed.

The diagnostic system 200 can include a monitor assembly 204 that is configured to be coupled to the pressure guidewire 208. The diagnostic system 200 may include a connection (indicated by the dashed line) that facilitates connection to and disconnection of the pressure guidewire 208 from the monitor assembly 204. The connection to and disconnection from the monitor assembly 204 is useful in allowing a clinician to use the pressure guidewire 208 initially for assessing the effect of the heart valve damage. The pressure guidewire 208 may also be used for delivering a treatment device such as a balloon catheter or valve delivery system.

A fiber optic interface cable 202 can be used to couple the pressure guidewire 208 with the monitor assembly 204 by way of a handle 207. In some embodiments, the system 200 receives an input from a tubular catheter body used to access the vasculature. For example, the access catheter 20 may be an access catheter. A distal tip of the pressure sensing of or in the access catheter 20 can be positioned adjacent the treatment site such that pressure signals corresponding to the pressure on a first side of the treatment site, e.g., in the aorta, can be obtained. This pressure measurement is sometimes referred to herein as Pa. In other configurations, the system 200 may include a pressure sensing device, such as a pigtail catheter, delivered separate from the pressure guidewire to obtain Pa.

The pressure guidewire 208 can take any suitable form. For example, the pressure guidewire 208 may include a proximal segment that has a proximal end that is positioned outside the patient and a distal end that may be advanced through the access catheter 20 to the vasculature. The pressure guidewire 208 can be configured to have the flexibility to navigate the tortuous vasculature while maintaining structural integrity for pushability and torqueability. For example, at least proximal section of the pressure guidewire 208 may be supported by a connector tube and/or core for structural integrity, while a distal section of the pressure guidewire 208 can be formed to include an atraumatic curvature 250, such as the coiled end shown in Figure 2B, to provide more flexibility and prevent puncture. In other configurations, a curved distal section may be joined to the pressure guidewire 208 to provide the atraumatic curvature 250.

Any sensing modality can be used. For example, an optical sensor can be configured to sense pressure when exposed to blood. The optical sensor can be disposed within an interior space of the pressure guidewire 208 in fluid communication with an exterior of the pressure guidewire 208. The sensor may be an optical or electrical pressure sensor. The sensor can be selectively placed in communication with the monitor assembly 204 by pressure wire lead(s) disposed between the sensor and a proximal end of the pressure guidewire 208. The pressure wire lead(s) may be an optical fiber or an electrical wire.

As shown in Figure 2B, the pressure sensor may be located anywhere along the distal section of the pressure guidewire 208. For example, the pressure sensor may be positioned near the distal-most tip of the guidewire at location 206D, along the curvature 250 of the guidewire at location 206C, at the transition to the curvature 250 of the guidewire at location 206B, or proximal of the curvature 250 of the guidewire at location 206A. For example, location 206C may be at about 270 degrees around the curvature 250 from the straight region (around location 206A) of the pressure guidewire and location 206D may be about 540 degrees around the curvature from the straight region of the pressure guidewire. However, the pressure sensor may be located any position in the curved distal region of the pressure guidewire, for example between and including about 0 degrees to about 90 degrees, between and including about 90 degrees to about 180 degrees, between and including about 180 degrees to about 270 degrees, between and including about 270 degrees to about 360 degrees, between and including about 360 degrees to about 450 degrees, or between and including about 450 degrees to about 540 degrees from the straight region of the pressure guidewire.

When the distal section is curled up, pressure sensor may be positioned about 270 degrees along the curvature 250 from the straight section of the pressure guidewire 208. The location of the pressure sensor within the distal section of the guidewire may influence the accuracy of the pressure measurements. For example, when the pressure sensor is in the more distal locations 206C, 206D, the pressure sensor may be more centrally located within the chamber of the heart, e.g. the left ventricle LV, and displaced from the chamber walls. Also, in the more distal locations 206C, 206D the pressure sensor is less likely to be obstructed by the access catheter or other delivery catheter during the valvuloplasty or heart replacement procedure. In the more proximal positions 206A, 206B, the pressure measurements will be taken closer to the heart valve but it is possible to perform equalization in the aorta A while maintaining the distal tip of the pressure guidewire 208 within the left ventricle LV. In some procedures, performing equalization in the aorta A requires less manipulation of the pigtail catheter or other pressure sensing device is required. For example, during an aortic valve procedure, the pigtail catheter is already located in the aorta. Although the pressure sensor may be proximal to the curvature 250, the pressure sensor is sufficiently distal to take pressure measurements distal to the heart valve. Leaving the distal tip of the pressure guidewire 208 within the left ventricle LV maintains access to the left ventricle LV.

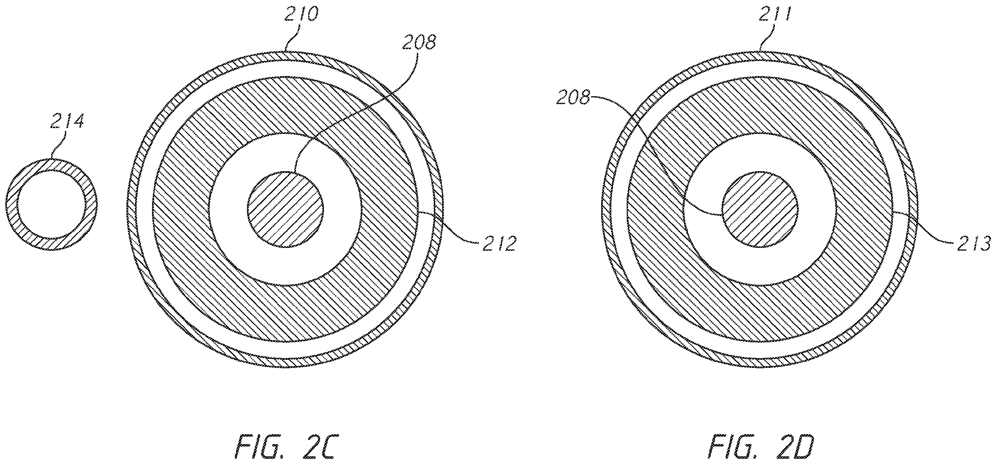

Figure 2C illustrates a cross-section of a TAVR system within a descending aorta of a patient, with the anatomy removed for clarity. The TAVR system could be used in connection with the monitor display 204. For example, the pressure guidewire 208 extends through an access catheter 210. The same access catheter 210 can be used to advance a delivery system 212 over the pressure guidewire 208. The delivery system 212 may be used to advance a valve replacement or other treatment device. Other configurations are also possible. For example, the catheter for the access catheter 210 could be exchanged with the delivery system 212 and thereafter advanced over the pressure guidewire 208. As shown, the pressure sensing device that is used to provide pressure signals for the pressure of blood in the aorta is an aortic pigtail catheter 214 delivered separately from the access catheter 210, although possibly from the same access point.

In other configurations, the access catheter 210 or the delivery system 212 may be used to obtain pressure signals for the pressure of blood in the aorta and thus may be the pressure sensing device for aortic pressure. As shown in Figure 2D, for a mitral valve replacement, the access catheter 211 may be the pressure sensing device. The pressure guidewire 208 extends through the access catheter 211 and the delivery system 213 may be advanced over the pressure guidewire 208. The delivery system 213 may be used to deliver a mitral valve or other replacement or treatment device.

As explained above, the pressure guidewire may be used to induce a rapid heart beat, e.g., by rapid pacing. A current may be delivered through a conductor in the guide wire, e.g., through the core wire, to the anatomy. The pressure guidewire may include an insulator along at least a portion or along portions of the pressure guidewire, for example a polymeric layer such as PTFE layer, to insulate the patient from the current. One or more portions electrically conductive portions of the pressure guidewire (e.g., a core wire or conductive connector tube) may be exposed through the insulator to allow a pacemaker or other electrical impulse generator to be attached to the electrically conductive portion of the pressure guidewire. The one or more exposed portions may be located anywhere along the length of the pressure guidewire, for example at a proximal end of the pressure guidewire and/or spaced apart from the proximal end of the pressure guidewire.

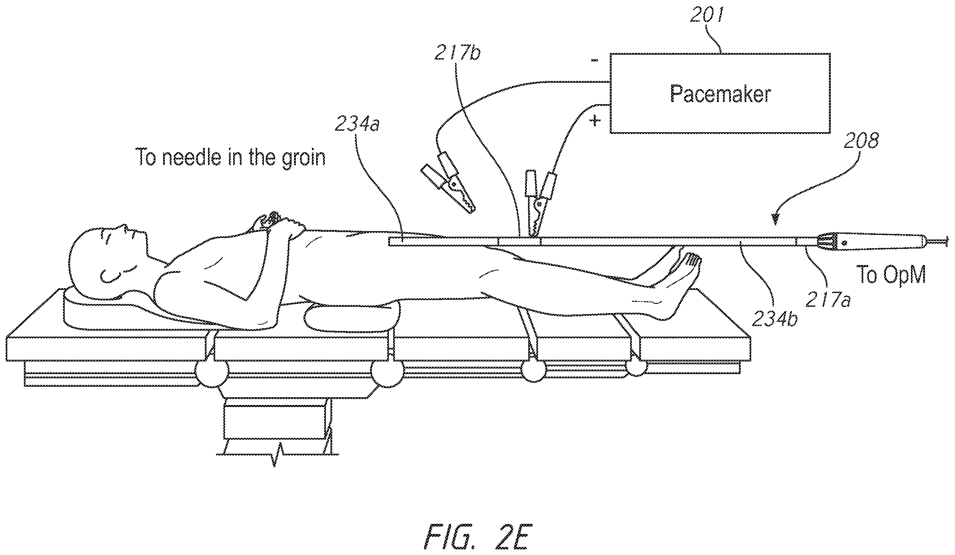

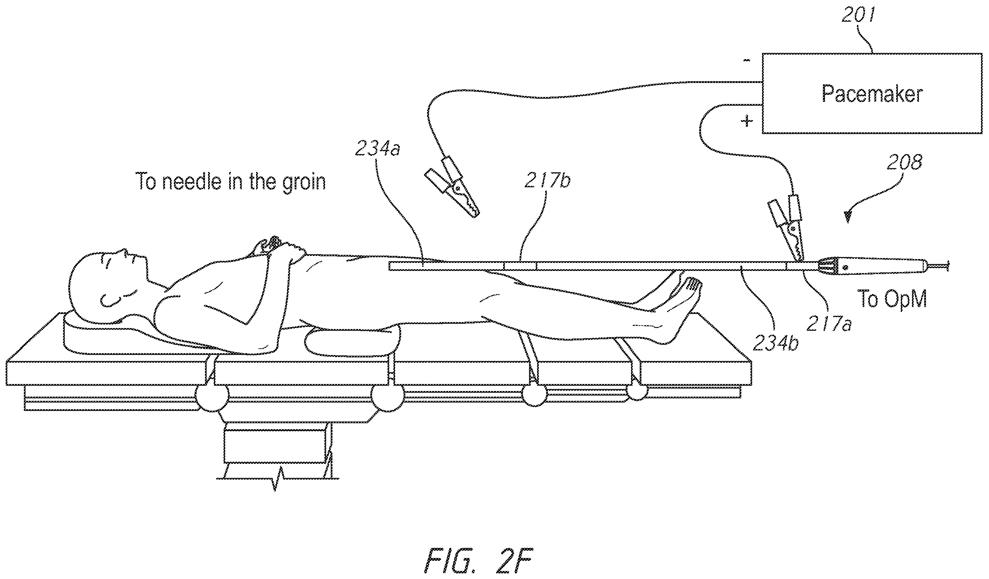

For example, as shown in Figure 2E, the core wire (or other electrically conductive portion) may be exposed from the insulator at least at a first conductive section 217a and a second conductive section 217b spaced apart from the first conductive section 217a. The first conductive section 217a may be located at a proximal portion of the pressure guidewire or closer to the proximal portion of the pressure guidewire than the distal portion of the pressure guidewire. The second conductive section 217b may be positioned between the distal end of the pressure guidewire and the first conductive section 217a. The pressure guidewire 208 may include a fewer or a greater number of exposed conductive sections. Viewed another way, the pressure guidewire may include at least a first insulator section 234a and a second insulator section 234b spaced apart from the first insulator section 234a. The first insulator section 234a may be positioned at a distal portion of the pressure guidewire or closer to the distal end of the pressure guidewire compared to the proximal end of the pressure guidewire. The second insulator section 234b may extend between the first insulator section 234a and the proximal end of the pressure guidewire. The multiple, exposed conductive sections 217a, 217b provide a physician with the option of connecting the pacemaker 201 closer to the proximal end of the pressure guidewire 208 (Figure 2F) or closer to a distal end of the pressure guidewire (Figure 2E). One of the exposed conductive sections may be more conveniently located compared to the other exposed conductive sections. For example, a clinician may desire to connect the pacemaker to an exposed conductive section closer to the distal end of the pressure guidewire when the pacemaker connection needs to be placed near the groin. On the other hand, a clinician may prefer to connect the pacemaker to an exposed conductive section closer to the proximal end of the pressure guidewire to avoid possible interference with the delivery system.

The rapid pacing features described with respect to Figures 2E and 2F may be applied to any of the pressure guidewires described herein.

### a. WIRE-BASED PRESSURE GUIDEWIRES

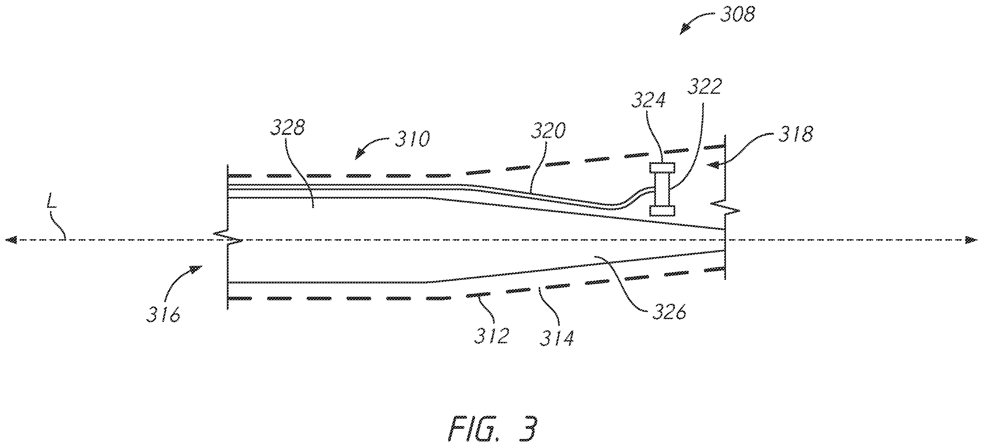

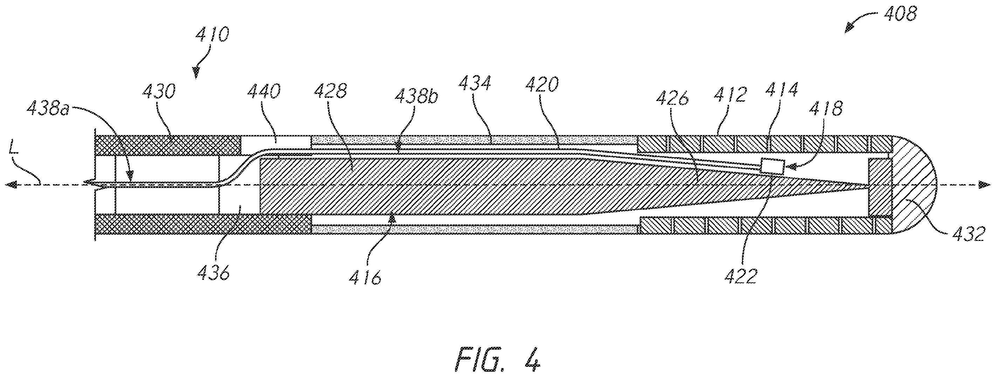

Figures 3 and 4 illustrate different pressure guidewires 308, 408 that may be used in any of the above-described methods. Numerals used to identify features of the pressure guidewire 308 are incremented by a factor of one hundred (100) to identify like features of the pressure guidewire 408. This numbering convention generally applies to the remainder of the figures. Any component of the pressure guidewires described herein can be interchanged.

In general, the pressure guidewires 308, 408 include an outer tube 310, 410 defining a lumen, a core wire 316, 416 extending at least partially through the lumen of the outer tube 310, 410, a pressure sensor assembly 318, 418 disposed within the lumen of the outer tube 310, 410, and/or a distal tip 432. The pressure guidewire 308 also can include a distal tip that can be the same as or similar to the tip 432 or any of the other tips disclosed herein. An outer diameter of the pressure guidewire 308, 408 may be uniform or substantially uniform along substantially the entire or entire working length of the pressure guidewire 308, 408. For example, the outer diameter of the pressure guidewire 308 may be uniform or substantially uniform along the entire working length, excluding distal tip 432 or atraumatic curvature 250. The pressure guidewire 308, 408 may include an outer diameter of up to 0.035 inches, for example between 0.018 inches and 0.035 inches. In some configurations, the distal portion of the pressure guidewire 308, 408 may be form an atraumatic curvature 250 such as the coiled portion shown in Figure 2B. In other configurations, the distal portion of the pressure guidewire 308, 408 may remain straight from at least the pressure sensor of the pressure sensor assembly to the distal tip of the pressure guidewire.

Figure 3 is a schematic view of one variation of the pressure sensing guidewire 308. As illustrated, at least a distal portion of the outer tube 310 may be coiled. For example, the coil portion 312 may be a flat ribbon coil or a round coil. The coil portion 312 may extend along a majority of the working length of the pressure guidewire 308, along substantially the entire working length of the pressure guidewire 308, or along the entire working length of the pressure guidewire 308. With a substantial length of the outer tube 310 being coiled, the coil portion 312 provides sufficient flexibility and softness to avoid any trauma during use (e.g. perforation and/or dissection). The coil portion 312 also promotes safety in case of distal tip failure. When rapid pacing, the coil portion 312 may also ensure electrical contact with the heart.

As shown in Figure 3, at least a proximal portion 328 of the core wire 316 may be concentric with the outer tube 310 and extend through at least a portion of the lumen of the outer tube 310. For example, the core wire 316 may extend along a majority of the working length of the pressure guidewire 308, along substantially the entire working length of the pressure guidewire 308, or along the entire working length of the pressure guidewire 308. The core wire 316 provides the pressure guidewire 308 with sufficient rigidity for pushability and to prevent kinking. It also provides sufficient rigidity to support the delivery catheter during valve implementation.

At least a portion of the core wire 316 may include a reduced diameter portion 326 to provide space in the lumen outer tube 310 for a pressure sensor 322. For example, as shown in Figure 3, the reduced diameter portion 326 may be tapered toward the distal end of the pressure guidewire 308. The transition between the proximal portion 328 and the reduced diameter portion 326 of the core wire 316 may be positioned proximal of at least a portion or the entirety of the atraumatic curvature 250 in the distal section of the pressure guidewire 308 (shown in Figure 2B) to promote a flexible transition to the atraumatic curvature 250 of the pressure guidewire 308. The core wire 316 would continue to extend through at least a portion of the atraumatic curvature 250. This flexible transition acts as a force absorber and ensures no kink is formed in the proximal section of the atraumatic curvature 250 of the pressure guidewire 308. A kink could complicate a procedure, such as advancing another catheter over the guidewire 308 or removing the guidewire 308 from the patient without trauma.

The proximal portion 328 of the core wire 316 may include an outer diameter of up to 0.03 inches, for example between 0.015 inches and 0.03 inches. A reduced diameter portion 326 of the core wire 316 may include an outer diameter that is less than one-third, or less than one-fourth, of the outer diameter of the proximal portion 328 of the core wire 316. For example, the reduced diameter portion 326 of the core wire 316 may include an outer diameter of less than 0.01 inches or less than 0.0075 inches.

The core wire 316 may include a conductive material such as stainless steel to provide a conductive path for current applied to the guidewire 308 in connection with a rapid pacing technique as described above. A proximal end of the core wire 316 may be exposed from the proximal end of the outer tube 310 for connection to the monitor display 204 and/or connection to a current generator. Less than ten percent, or less than five percent, of a length of the core wire 316 may be exposed from the proximal end of the outer tube 310 for connection to a current source for rapid pacing.

The pressure sensor assembly 318 may include a pressure sensor 322 and one or more pressure wire leads 320 extending from the pressure sensor 322. The pressure wire leads 320 may extend along the core wire 316. For example, the pressure sensor 322 may be an optical or electrical sensor, membrane-based sensor, a MEMS sensor or other device that can generate a signal in response to pressure levels or fluctuations. The one or more pressure wire leads 320 may be an optical fiber or electrical wire. As shown in Figure 3, the pressure sensor assembly 318 may also include a sensor housing 324 disposed over the pressure sensor 322 and positioned between the outer tube 310 and the core wire 316. The sensor housing 324 can include a ring or short tubular member or a cylinder in which a membrane is supported. The sensor housing 324 can enhance handling during assembly in the coil portion 312.

The pressure sensor assembly 318 may be disposed radially between the core wire 316 and the outer tube 310 with the pressure sensor 322 disposed radially between the reduced diameter portion 326 of the core wire 316 and the coiled portion 312 of the outer tube 310. At least a portion of the pressure sensor assembly 318 may be off-axis relative to a longitudinal axis L of the pressure guidewire 308. In some configurations, the entire pressure sensor assembly 318 may be off-axis relative to the longitudinal axis of the pressure guidewire 308.

The pressure sensor 322 may be exposed to blood or other fluid through the spacing or gaps 314 in the coil portion 312. Although, in other variations, the outer tube 310 may include a sensor housing section with one or more openings to expose the pressure sensor 322 to blood or other fluid. The sensor housing section may be stiffer than the remainder of the coil portion 312. For example, the sensor housing section may be a metallic tube splitting the coil portion 312 into two sections. The sensor housing section may be mounted to a distal portion of a first coil section of the coil portion 312 and to a proximal portion of a second coil section of the coil portion 312. As another example, the coil portion 312 may include two coils welded together to create a stiffened section.

At least a portion of the pressure guidewire 308 may be covered by a lubricious insulator, for example a polymeric layer such as PTFE. The insulator may secure one or more pressure wire(s) lead(s) 320 in place. When rapid pacing is induced through the core wire 316, the insulator may also electrically isolate the core wire 316 from the patient along the length of the insulator. The insulator may replace the need for a separate catheter to electrically isolate the pressure guidewire 308.

Figure 4 illustrates another variation of the pressure guidewire 408. The pressure guidewire 408 can include any of the features described with respect to the pressure guidewire 308. In this variation, a distal portion of the outer tube 410 may be formed by the coil portion 412. For example, the coil portion 412 may be a flat ribbon coil or a round coil. A proximal portion of the outer tube 410 may be formed by a connector tube 430. The connector tube 430 may include a conductive material to facilitate rapid pacing. For example, the connector tube 430 may be formed with a metal structure such as a stainless steel tube. The connector tube 430 is not covered with a coating or other insulator to allow for rapid pacing. In some configurations, current may alternatively or additionally flow through the one or more pressure wire leads 420. The connector tube 430 may be connected directly or indirectly to the coil portion 412 and/or to the distal tip 432. For example, the coil portion 412 may be indirectly connected to the connector tube 430 by an insulated portion. The insulated portion can provide a length that is insulated from the patient and thus may be an insulator portion 434 in some embodiments. The insulator portion 434 may insulate the patient from the core wire 416. In some configurations, the insulator portion 434 may include a polymeric layer such as PTFE.

At least a non-reduced diameter portion of the core wire 416 may be concentric with the outer tube 410. The core wire 416 may extend through at least the coil portion 412, but may also extend through at least a portion of the insulator portion 434 and/or the connector tube 430 of the outer tube 410. For example, a proximal end of the core wire 416 may be sealed, for example using adhesive 436, to a distal end of the connector tube 430 and extend distally from a distal end of the connector tube 430.

The core wire 416 may include any of the features of the core wire 316. For example, a distal portion of the core wire 416 may include a reduced diameter portion 426. A proximal end of the coil portion 412 may be distal of a transition between the non-reduced diameter portion 428 and the reduced diameter portion 426 of the core wire 416.

The pressure sensor assembly 418 may be disposed radially between the core wire 416 and the outer tube 410 with the pressure sensor 422 positioned radially between the reduced diameter portion 426 of the core wire 416 and the coil portion 412. At least a portion of the pressure sensor assembly 418 may be off-axis relative to a longitudinal axis L of the pressure guidewire 408. For example, a first section 438a of at least one pressure wire lead 420 may be concentric with the outer tube 410 and a second section 438b of the pressure wire lead 420 may be off-axis relative to a longitudinal axis of the outer tube 410. The outer tube 410 may include an opening 440 to permit the pressure wire lead 420 to transition from the first section 438a that is concentric with the outer tube 410 to the second section 438b that is off-axis relative to the longitudinal axis of the outer tube 410. The opening 440 may be a partial thickness cut out or extend through the full thickness of the outer tube 410. If the opening 430 extends through the full thickness of the outer tube 410, the opening 440 may be sealed, for example with adhesive 436, to prevent blood or other fluids from flowing into the pressure guidewire through the opening 440. As shown in Figure 4, the opening 440 is disposed in the connector tube 430. However, in other configurations, the opening 440 may be disposed in the insulator portion 434.

Alternative to the opening 440, the core wire 416 may be sized or offset relative to a longitudinal axis of the pressure guidewire 408 to permit the pressure wire lead 420 to transition from the first section 438a that is concentric with the outer tube 410 to the second section 438b that is off-axis relative to the longitudinal axis of the outer tube 410. The core wire 416 can have a groove in one side configured to receive a span of the pressure wire lead 420 such that the lead can transition from the first section 438a to the second section 438b.

The pressure guidewire 408 may include a distal tip 432 that is rounded to form an atraumatic tip. For example, the distal tip 432 may have a hemispherical shape. The tip 432 may also reduce or even to prevent ingress of unwanted foreign matter through a distal end of the pressure guidewire 408.

In some configurations, the distal tip 432 is a separate component adhered, welded, and/or otherwise joined to the coil portion 412 and/or the core wire 416. The distal tip may be joined to an inner surface of the coil portion 412 and/or the distal most edge of the coil portion 412. The core wire 416 may be bent up to 180 degrees within the outer tube 410 to strengthen the adhesive joint to the distal tip 432. In other configurations, the distal tip 432 may be an enlarged distal end of the core wire 416 that is distal of the reduced diameter portion 426. The distal end of the core wire 416 may be adhered, welded, and/or otherwise joined to the inner surface and/or distal most edge of the coil portion 412. In one method, the distal tip 432 is formed by transforming an enlarged segment of the core wire 416 into a hemispherical member. The enlarged segment can be melted to form the hemispherical member. The hemispherical member can be joined to a distal portion of the coil portion 412. In any of these configurations, the atraumatic portion of the distal tip 432 may be formed from the core wire 416, adhesive, and/or welding.

### b. TUBE-BASED PRESSURE GUIDEWIRES

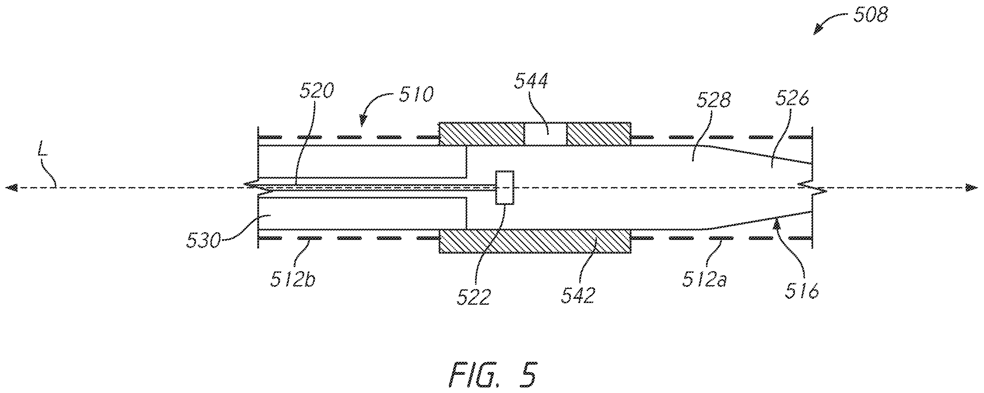

Figures 5 to 10C illustrate further variations of pressure guidewires that may be used in any of the above-described methods. The pressure guidewires described below may include any of the features of the above-described pressure guidewires 308, 408. In general, the pressure guidewires shown in Figures 5 to 10C include an outer tube defining a lumen, connector tube positioned radially inward of the outer tube, a pressure sensor assembly disposed within the lumen of the outer tube, and/or a distal tip. An outer diameter of the pressure guidewire may be uniform or substantially uniform along substantially the entire or entire working length of the pressure guidewire. For example, the outer diameter of the pressure guidewire may be uniform or substantially uniform along the entire working length, excluding distal tip or grinded down curvature. The pressure guidewire may include an outer diameter of up to 0.035 inches, for example between 0.018 inches and 0.035 inches. In some configurations, the distal portion of the pressure guidewire may be formed to an atraumatic curvature 250 as shown in Figure 2B. In other configurations, the distal portion of the pressure guidewire may remain straight.

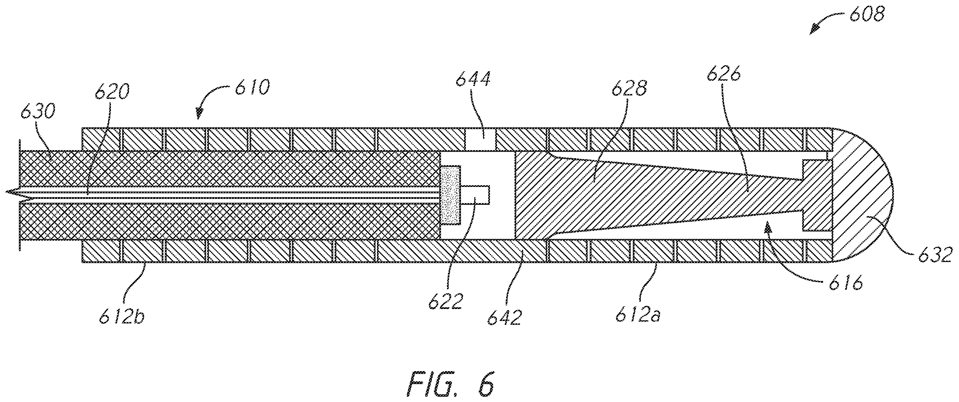

The connector tube may include an inner diameter that is less than one-third, or less than one-fourth an outer diameter of the connector tube. For example, the connector tube may include an outer diameter of up to 0.035 inches, for example between 0.018 inches and 0.035 inches, and an inner diameter of less than 0.01 inches, for example less than 0.007 inches. The connector tube may have a uniform outer diameter (see Figure 5) or a non-uniform diameter (see Figure 6). In the non-uniform configurations, a reduced diameter portion of the connector tube may have an outer diameter of less than or equal to about 0.027 inches. The connector tube may extend along a majority of or substantially the entire working length of the pressure guidewire. For example, the connector tube may extend at least eighty percent, or at least ninety percent, of the working length of the pressure guidewire.

The connector tube may be constructed of a conductive metal. For example, the connector tube may be a stainless steel tube. A proximal end of the connector tube may be exposed from the proximal end of the outer tube for connection to the monitor display and/or connection to a current generator. Thus, at least the proximal end of the connector tube may be uncoated.

The pressure guidewire may also include a core wire distal to the connector tube. In a venous or trans-apical aortic valve application, the portion with the core wire may be disposed in blood flow downstream of a portion with the connector tube. In an arterial or trans-apical mitral valve application, the portion with the core wire may be disposed in blood flow upstream of a portion with the connector tube. The core wire may include an outer diameter of up to 0.03 inches, for example between 0.018 inches and 0.03 inches. A reduced diameter portion of the core wire may include an outer diameter that is less than one-third, or less than one-fourth, of the outer diameter of the remainder of the core wire. For example, the reduced diameter portion of the core wire may include an outer diameter of less than 0.01 inches or less than 0.0075 inches. The core wire may extend along only a distal portion of the pressure guidewire, for example along less than twenty percent or less than ten percent or less than 5 percent of a working length of the pressure guidewire.

Figure 5 is a schematic view another variation of the pressure sensing guidewire 508. As illustrated, at least a distal portion of the outer tube 510 may be coiled. For example, the coiled portion may be a flat ribbon coil or a round coil. As shown in Figure 5, the coiled portion may include two coiled sections 512a, 512b separated from each other by a sensor housing 542. Together, the coil portions 512a, 512b may extend along a majority of the working length of the pressure guidewire 508 or along substantially the entire working length of the pressure guidewire 508. For example, together, the coiled portions 512a, 512b may extend at least eighty percent, or at least ninety percent, of the working length of the pressure guidewire 508. With a substantial length of the outer tube 510 being coiled, the coil portions 512a, 512b provides sufficient flexibility to navigate tortuous vasculature. The distal coil portion 512a also promotes safety in case of failure along a coiled portion, e.g., distal tip failure. When used for rapid pacing, the distal coil portion 512a may also ensures electrical contact with the inner walls of the patient's heart, e.g., with inner walls of the left ventricle.

As shown in Figure 5, at least a proximal portion 528 of the core wire 516 may be concentric with the outer tube 510 and extend through at least a portion of the lumen of the outer tube 510. The diameter of the proximal portion 528 of the core wire 516 may be the same as the outermost diameter of the connector tube 530. At least a portion of the core wire 516 may include a reduced diameter portion 526 such as a tapered portion that is tapered toward the distal end of the pressure guidewire 508. The transition between the proximal portion 528 and the reduced diameter portion 526 of the core wire 516 may be positioned proximal of the atraumatic curvature 250 in the distal section of the pressure guidewire 508 to promote a flexible transition to the atraumatic curvature 250 of the pressure guidewire 508. This flexible transition acts as a force absorber and ensures no kink is formed in the proximal section of the atraumatic curvature 250 of the pressure guidewire 508. A kink could complicate a procedure, such as advancing another catheter over the guidewire 508 or removing the guidewire 308 from the patient without trauma. The core wire 516 may include a conductive material such as stainless steel to provide rapid pacing as described above.

The pressure sensor assembly 518 may include a pressure sensor 522 and one or more pressure wires leads 520 extending from the pressure sensor 522. For example, the pressure sensor 522 may be an optical or electrical sensor, membrane-based sensor, or otherwise. The pressure wire(s) lead(s) 520 may be an optical fiber or electrical wires. The pressure wire(s) lead(s) 520 may extend through the lumen of the connector tube 530. The connector tube 530 locates the pressure wire(s) lead(s) 520 along the central longitudinal axis L of the pressure guidewire 508. The pressure wire(s) lead(s) 520 may be secured to and in some cases also sealed to the connector tube 530, for example using adhesive. In some cases, the adhesive provides a seal to prevent fluid from flowing proximally through the connector tube 530. Adhesive may also be used in the proximal end of the connector tube 530 to secure the optical fiber 520 concentrically to the connector tube 530.

As shown in Figure 5, the pressure sensor 522 may be disposed within the pressure sensor housing 542 of the outer tube 510. The sensor housing 542 protects the pressure sensor 522 but also provides a connection between the coil portions 512a, 512b. The pressure sensor 522 may be exposed to blood or other fluid through the at least one opening 544 in the sensor housing 542. As illustrated, the sensor housing 542 may be a metal tube joining the two coil portions 512a, 512b, but in other variations, the sensor housing 542 may be formed by welding several coils together to form a welded portion joining the coil portions 512a, 512b.

The sensor housing 542 and the pressure sensor 522 may be positioned proximal of the atraumatic curvature 250 shown in Figure 2B, for example at location 206A. However, as explained above, the pressure sensor may also be positioned anywhere along the curvature 250 in the distal section of the pressure guidewire 508.

At least a portion of the pressure guidewire 508 may be covered by a lubricious insulator, for example a polymeric layer such as PTFE. When rapid pacing is induced through the connector tube 530 and/or the core wire 516, the insulator may also electrically isolate portions of the pressure guidewire 508. The insulator may replace the need for a separate catheter body to electrically isolate the pressure guidewire 508.

Figure 6 is a cross-sectional view of another variation of the pressure sensing guidewire 608. The pressure sensing guidewire 608 is similar to the pressure sensing guidewire 508 except as described differently below. The disclosure in connection with Figure 6 can be seen to supplement that of Figure 5. The pressure sensing guidewire 608 includes a distal tip 632. The distal tip 632 is similar to the distal tip 432 except as described differently below. The distal tip 632 provide for atraumatic interaction with blood vessels, valves and heart wall chambers. The tip 632 also may reduce or prevent ingress of foreign matter, e.g., components or fluid, through a distal end of the pressure guidewire 608. The distal tip 632 may have a hemispherical shape.

In some configurations, the distal tip 632 is a separate component adhered, welded, and/or otherwise joined to the coil portion 612a and/or the core wire 616. The distal tip 632 may be joined to an inner surface of the coil portion 612a and/or the distal most edge of the coil portion 612a. The core wire 616 may be bent up to 180 degrees within the outer tube 610 to strengthen the adhesive joint to the distal tip 632. In other configurations, the distal tip 632 may be an enlarged distal end of the core wire 616 that is distal of the reduced diameter portion 626. The distal end of the core wire 616 may be adhered, welded, and/or otherwise joined to an inner surface and/or distal most edge of the coil portion 612a. In any of these configurations, the atraumatic portion of the distal tip 632 may be formed from the core wire 616, such as by melting or otherwise reforming an enlarged segment of the core wire 616 to create the desired shape.

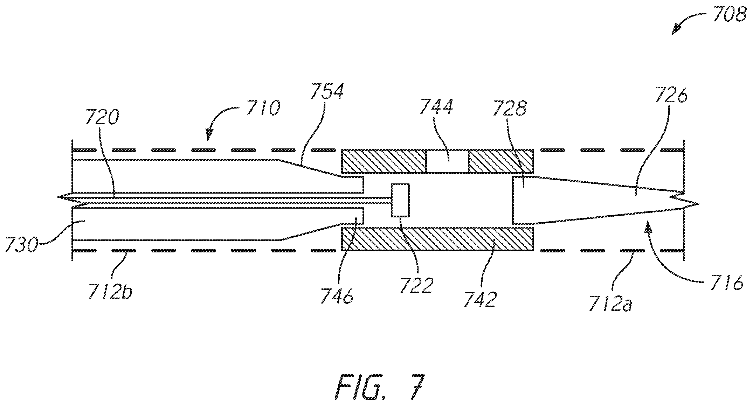

Figure 7 is a schematic view of another variation of the pressure sensing guidewire 708. The pressure sensing guidewire 708 is similar to the pressure sensing guidewire 508 except that sensor housing 742 and pressure sensor 722 may be positioned more distally into the distal curvature 250 of the pressure guidewire 708, for example at locations 206B or 206C shown in Figure 2B. However, as discussed above, it can be beneficial to reduce the diameter of the inner core wire to promote flexibility at the transition to the distal curvature 250. Thus, the sensor housing 742 and the pressure sensor 722 may be positioned in a region in which the connector tube 730 and/or core wire 716 have transitioned to a reduced diameter. For example, as shown in Figure 7, the connector tube 730 may have a reduced diameter section 746 at the distal end of the connector tube 730. Optionally, the connector tube 730 may be tapered toward the reduced diameter section 746 at tapered portion 754. A diameter of the proximal end of the core wire 716 may also be less than an outermost diameter of the connector tube 730, e.g. at a distal end or distal region thereof. In this configuration, an outer diameter of the sensor housing 742 may also be reduced compared to the sensor housing 542. An outer diameter of a proximal portion of the connector tube 730 may be less than or equal to an inner diameter of the coil portion 712b and/or greater than an inner diameter of the sensor housing 742. As illustrated, the proximal portion of the connector tube 730 may be positioned within the coil portion 712b, while the reduced diameter section 746 may be positioned within the sensor housing 742. In this configuration, an inner diameter of coil portion 712a and/or coil portion 712b may be different from, e.g., larger than, an inner diameter of the sensor housing 742. An outer diameter of the coil portions 712a, 712b may be about the same as the outer diameter of the sensor housing 742. In other configurations, an inner diameter of the coil portion 712a and/or coil portion 712b may be the same as the inner diameter of the sensor housing 742 (similar to guidewire 608).

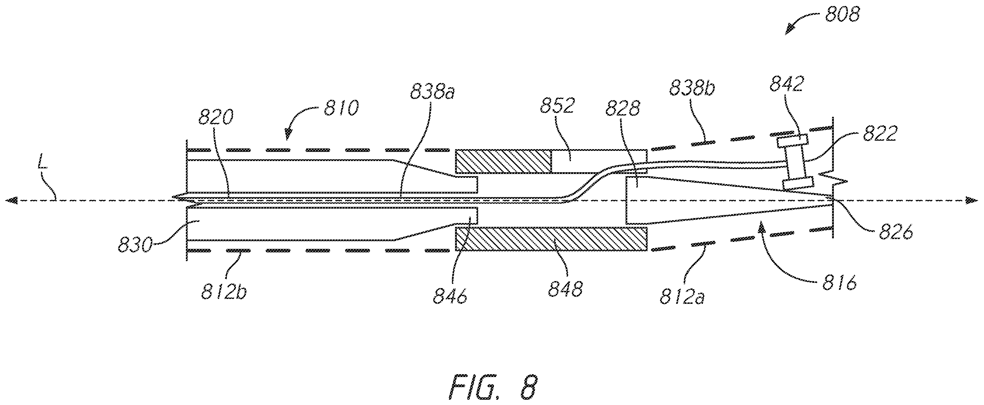

Figure 8 is a schematic view of another variation of the pressure sensing guidewire 808. The pressure sensing guidewire 808 is similar to the pressure sensing guidewire 708 except that sensor housing 842 and pressure sensor 822 may be positioned more distally into the distal curvature 250 of the pressure guidewire 808, for example at location 206D shown in Figure 2B. However, as discussed above, it can be beneficial to reduce the diameter of the inner core wire to promote flexibility at the transition to the distal curvature 250. Thus, in the region of 206D in the distal curvature 250, the reduced diameter portion 826 of the core wire 816 may have a sufficiently reduced diameter to permit the positioning of the sensor 822 radially between the distal coil portion 812a and the reduced diameter portion 826 of the core wire 816. As shown in Figure 8, the sensor 822 may have a separate sensor housing 842 positioned around the sensor 822.

Instead of a sensor housing along the outer tube 810, the pressure guidewire 808 includes a connector 848 extending between the coil portions 812a, 812b. The connector 848 may include an opening 852 to permit at least one pressure wire lead 820 to transition from the first section 838a that is concentric with the outer tube 410 and within the connector tube 830 to the second section 838b that is off-axis relative to the longitudinal axis L of the outer tube 810. The opening 840 may be a partial thickness cut out or extend through the full thickness of the outer tube 810. If the opening 830 extends through the full thickness of the outer tube 810, the opening 840 may be sealed, for example with adhesive, to prevent fluid from flowing into the pressure guidewire through the opening 840.

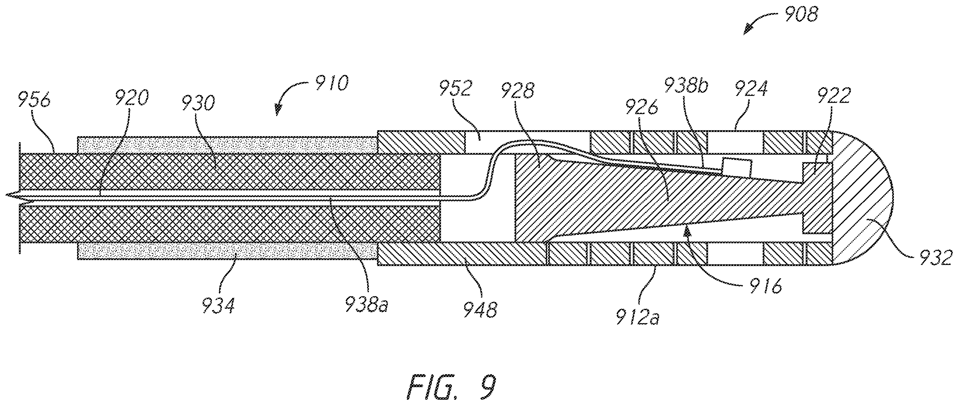

Figure 9 is a cross-sectional view of another variation of the pressure sensing guidewire 908. The pressure sensing guidewire 908 is similar to the pressure sensing guidewire 808 except that except that Figure 9 includes distal tip 932. The distal tip 932 may include any of the features of the distal tip 632 shown in Figure 6.

The outer tube 910 includes an insulator portion 934 and a coil portion 912 joined by the connector 948. The coil portion 912 may be a flat ribbon coil or a round coil. The insulator portion 934 surrounds at least a portion of the connector tube 930. The insulator portion 934 may include a polymeric layer such as PTFE to electrically isolate the connector tube 930 from the patient during rapid pacing. A proximal end 956 of the connector tube 930 may be exposed from the proximal end of the insulator portion 934 for connection to the monitor and/or connection to a current generator. Thus, at least the proximal end of the connector tube 930 may be uncoated.

As illustrated, the connector 948 may be a metal tube joining the insulator portion 934 and the coil portion 912, but in other variations, the connector 948 may be a welded portion joining the insulator portion 934 and the coil portion 912.

The one or more pressure wires leads 920 may be sealed to the inner lumen of the connector tube 930, for example using adhesive, to prevent fluid from flowing proximally and ensuring concentricity of the optical fiber for signal transmission.

The pressure sensor 922 may be exposed to blood or other fluid through the spacing or gaps in the coil portion 912. The outer tube 910 may also include sensor housing section 924. The sensor housing section may be stiffer than the remainder of the coil portion 912. For example, the sensor housing section 924 may be a metallic tube splitting the coil portion 912 into two sections. The sensor housing section 924 may be mounted to a distal portion of a first coil section of the coil portion 912 and to a proximal portion of a second coil section of the coil portion 912. The sensor housing section 924 may include one or more openings to expose the pressure sensor 922 to blood or other fluid. As another example, the coil portion 912 may include two coils welded together to create a stiffened section that serves as the sensor housing section 924.

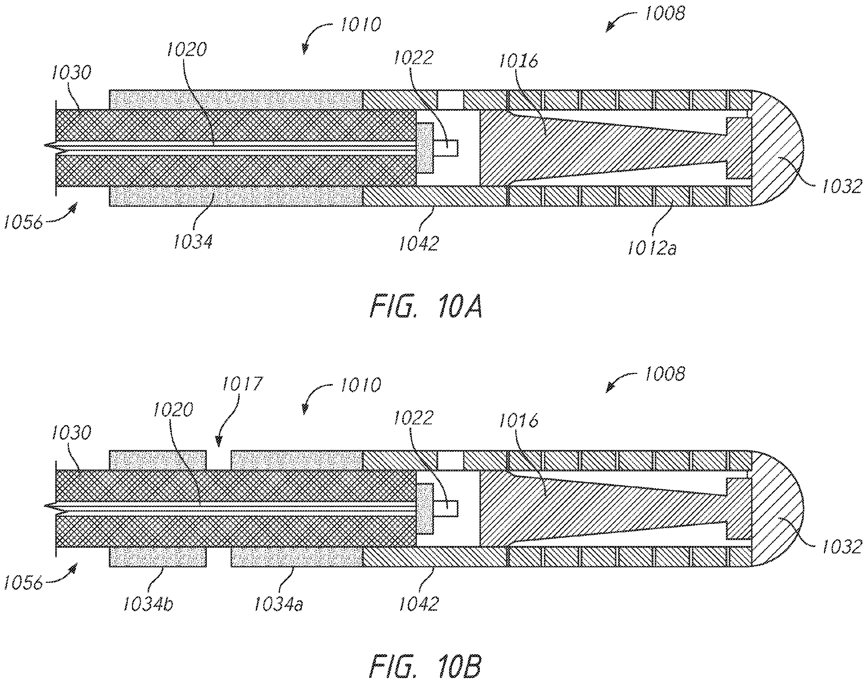

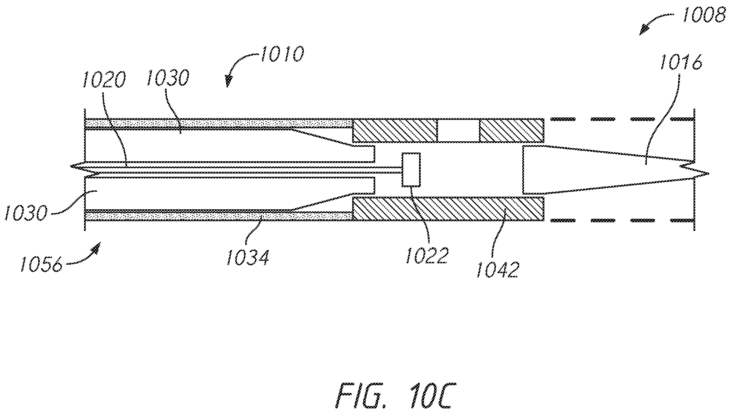

Figures 10A-10C illustrate cross-sectional views of variations of the pressure sensing guidewire 1008. The pressure sensing guidewire 1008 is similar to the pressure sensing guidewire 608 or 708 except that an insulator portion 1034 may surround at least a portion of the conductive connector tube 1030 and/or the pressure wire lead(s) 1020. As shown in Figures 10A and 10B, the connector tube 1030 may have a uniform diameter. However, in other configurations (see Figure 10C), a distal portion of the connector tube 1030 may have a reduced diameter. Optionally, the connector tube 1030 may be tapered toward the distal portion of the connector tube 1030. The insulator portion 1034 may form at least a portion of the outer tube 1010. For example, as shown in Figure 10A, the insulator portion 1034 may extend proximally from the sensor housing 1042 toward a proximal end 1056 of the pressure guidewire 1008. The proximal end 1056 of the connector tube 1030 may be exposed from the insulator portion 1034 for connection to the monitor and/or connection to a current generator for rapid pacing. The insulator portion 1034 electrically insulates portions of the pressure guidewire 1008 to allow for rapid pacing as described above. The insulator portion 1034 also provides lubricity for the delivery system to be provided over the pressure guidewire 1008. The insulator portion 1034 may include a polymeric layer such as PTFE. As shown in Figures 10A and 10B, an inner diameter of the insulator portion 1034 may be about the same as the inner diameter of the sensor housing 1042 and/or an outer diameter of the connector tube 1030 may be less than or equal to an inner diameter of the sensor housing 1042. However, in other configurations (see Figure 10C), an inner diameter of the insulator portion 1034 may be different from, e.g., greater than, an inner diameter of the sensor housing 1042. An outer diameter of the insulator portion 1034 may be about the same as an outer diameter of the sensor housing 1042. An outer diameter of a proximal portion of the connector tube 1030 may be less than or equal to an inner diameter of the insulator portion 1034 and/or greater than an inner diameter of the sensor housing 1042. An outer diameter of a distal portion of the connector tube 1030 may be less than or equal to the inner diameter of the sensor housing 1042. Optionally, the connector tube 1030 may include a tapered portion between the proximal portion and the distal portion of the connector tube 1030. The proximal portion of the connector tube 1030 may be positioned within the insulator portion 1034, while the distal portion of the connector tube 1030 may be positioned within the sensor housing 1042. A coil portion may surround the core wire 1016. An inner diameter of the coil portion may be the same as or different from, e.g., greater than, and inner diameter of the sensor housing 1042. An outer diameter of the coil portion may be about the same as the sensor housing 1042.

As shown in Figure 10A, the insulator portion 1034 may extend continuously from the sensor housing 1042 to the exposed proximal end 1056 of the pressure guidewire 1008. However, as shown in Figure 10B, an electrically conductive portion 1017 of the pressure guidewire 1008 may be exposed from the insulator portion 1034 to allow for a different connection point for the current generator, as described above in connection with Figures 2E-2F. The electrically conductive portion 1017 may be distal of the exposed proximal end 1058, for example proximal of and/or spaced apart from the sensor housing 1042. For example, the electrically conductive portion 1017 may be positioned between the proximal and distal ends of the insulator portion 1034. As illustrated, the electrically conductive portion 1017 is a portion the connector tube 1030; however, the electrically conductive portion 1017 may expose a portion of the core wire 1026 or any other conductive component of the pressure guidewire 1008. The electrically conductive portion 1017 may be in addition or in alternative to the exposed proximal end 1058. Additional electrically conductive portions 1017 may be exposed along a length of the pressure guidewire 1008.

### Terminology

As used herein, the relative terms "proximal" and "distal" shall be defined from the perspective of the user of the system. Thus, proximal refers to the direction toward the user of the system and distal refers to the direction away from the user of the system.

As used herein, the relative terms "upstream" and "downstream" shall be defined from the perspective of blood flow. Thus, downstream refers to the direction toward the aorta relative to the left ventricle.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments.

The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

The terms "approximately," "about," "generally," and "substantially" as used herein represent an amount close to the stated amount that still performs a desired function or achieves a desired result. For example, the terms "approximately," "about," "generally," and "substantially" may refer to an amount that is within less than 5% of the stated amount, as the context may dictate.

The ranges disclosed herein also encompass any and all overlap, sub-ranges, and combinations thereof. Language such as "up to," "at least," "greater than," "less than," "between" and the like includes the number recited. Numbers preceded by a term such as "about" or "approximately" include the recited numbers. For example, "about four" includes "four."

Any methods disclosed herein need not be performed in the order recited. The methods disclosed herein include certain actions taken by a practitioner; however, they can also include any third-party instruction of those actions, either expressly or by implication. For example, actions such as "distally moving a locking element" include "instructing distal movement of the locking element."

Although certain embodiments and examples have been described herein, it will be understood by those skilled in the art that many aspects of the humeral assemblies shown and described in the present disclosure may be differently combined and/or modified to form still further embodiments or acceptable examples. All such modifications and variations are intended to be included herein within the scope of this disclosure. A wide variety of designs and approaches are possible. No feature, structure, or step disclosed herein is essential or indispensable.

Some embodiments have been described in connection with the accompanying drawings. However, it should be understood that the figures are not drawn to scale. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein. Additionally, it will be recognized that any methods described herein may be practiced using any device suitable for performing the recited steps.

For purposes of this disclosure, certain aspects, advantages, and novel features are described herein. It is to be understood that not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

Moreover, while illustrative embodiments have been described herein, the scope of any and all embodiments having equivalent elements, modifications, omissions, combinations (e.g., of aspects across various embodiments), adaptations and/or alterations as would be appreciated by those in the art based on the present disclosure. The limitations in the claims are to be interpreted broadly based on the language employed in the claims and not limited to the examples described in the present specification or during the prosecution of the application, which examples are to be construed as non-exclusive. Further, the actions of the disclosed processes and methods may be modified in any manner, including by reordering actions and/or inserting additional actions and/or deleting actions. It is intended, therefore, that the specification and examples be considered as illustrative only, with a true scope and spirit being indicated by the claims and their full scope of equivalents.

**The** present application discloses subject matter in accordance with the following numbered clauses:
Clause 1. A guidewire comprising:
   a connector tube extending from a proximal end of the guidewire, the connector tube comprising a tube wall and a lumen extending therethough;
   a core wire extending distally of a distal end of the connector tube;
   a sensor assembly comprising:
      a sensor;
      a sensor housing positioned over the sensor; and
   an insulator portion surrounding at least a portion of the connector tube and extending proximally of the sensor housing, the insulator portion having a proximal end and a distal end.
Clause 2. The guidewire of Clause 1, wherein the connector tube comprises a conductive material.
Clause 3. The guidewire of Clause 1, wherein at least one conductive section of the connector tube is exposed from the insulator portion, and wherein, in use, the at least one conductive section is located outside of a patient.
Clause 4. The guidewire of Clause 3, wherein the at least one conductive section comprises a first conductive section a second conductive section, the second conductive section being spaced apart from the first conductive section.
Clause 5. The pressure guidewire of Clause 4, wherein the first conductive section is located at a proximal end of the guidewire and the second conductive section is located distal of the first conductive section.
Clause 6. The pressure guidewire of Clause 5, wherein the second conductive section is positioned between the proximal end of the insulator portion and the distal end of the insulator portion.
Clause 7. The guidewire of Clause 1, wherein the sensor is a pressure sensor.
Clause 8. The guidewire of Clause 1, further comprising a coil portion at least partially surrounding the core wire.
Clause 9. The guidewire of Clause 8, wherein the insulator portion and the coil portion have a same outer diameter.
Clause 10. The guidewire of Clause 1, wherein a distal portion of the connector tube is tapered.
Clause 11. The guidewire of Clause 1, wherein the core wire comprises a reduced diameter portion.
Clause 12. The guidewire of Clause 1, wherein the sensor is coaxial with the core wire. Clause 13. The guidewire of Clause 1, further comprising a distal tip at a distal end of the guidewire.
Clause 14. The guidewire of Clause 1, further comprising a wire lead extending from the sensor toward a proximal end of the guidewire, the wire lead at least partially extending through the lumen of the connector tube.
Clause 15. The guidewire of Clause 1, wherein the wire lead is sealed to the connector tube to prevent fluid from flowing proximally to a proximal end of the guidewire.
Clause 16. A pressure guidewire comprising:
   an outer tube comprising a sensor housing and a coil portion;
   connector tube positioned radially inward of the outer tube, the connector tube comprising a tube wall and a lumen extending therethough;
   a core wire positioned radially inward of the outer tube and longitudinally spaced apart from a distal end of the connector tube, the core wire comprising a reduced diameter portion;
   a pressure sensor assembly comprising a pressure sensor and a pressure wire lead extending from the pressure sensor toward a proximal end of the pressure guidewire, the pressure sensor being distal of the distal end of the connector tube, the pressure wire lead at least partially extending through the lumen of the connector tube; and
   a distal tip at a distal end of the outer tube.
Clause 17. The pressure guidewire of Clause 16, wherein the outer tube comprises a uniform diameter.
Clause 18. The pressure guidewire of Clause 16, wherein the pressure wire lead is sealed to the connector tube to prevent fluid from flowing proximally to a proximal end of the guidewire.
Clause 19. The pressure guidewire of Clause 16, wherein the pressure sensor is positioned radially between the outer tube and the core wire.
Clause 20. The pressure guidewire of Clause 16, wherein the connector tube comprises a conductive material.
Clause 21. The pressure guidewire of Clause 16, wherein at least a portion of the pressure wire lead is offset from a longitudinal axis of the connector tube.
Clause 22. The pressure guidewire of Clause 21, wherein the pressure wire lead is configured to transition from a first section that is concentric with the connector tube and a second section comprising the portion of the pressure wire lead that is offset from the longitudinal axis of the connector tube.
Clause 23. The pressure guidewire of Clause 16, wherein the pressure sensor is positioned within the coil portion.
Clause 24. The pressure guidewire of Clause 16, wherein the outer tube further comprises a sensor housing having at least one opening.
Clause 25. The pressure guidewire of Clause 24, wherein the at least one opening allows fluid to flow in the space between the connector tube and the core wire.
Clause 26. The pressure guidewire of Clause 24, wherein the pressure sensor is positioned within the sensor housing.
Clause 27. The pressure guidewire of Clause 24, wherein the pressure guidewire comprises a second coil portion extending proximally from the sensor housing.
Clause 28. The pressure guidewire of Clause 27, wherein the second coil portion extends from the sensor housing toward a proximal end of the pressure guidewire.
Clause 29. The pressure guidewire of Clause 16, wherein the outer tube further comprises an insulator portion extending proximally of the coil portion.
Clause 30. The pressure guidewire of Clause 16, wherein the distal tip is adhered to the distal section of the core wire.
Clause 31. The pressure guidewire of Clause 16, wherein the distal tip is welded to the coil portion.
Clause 32. The pressure guidewire of Clause 16, wherein the distal tip is an enlarged distal end of the core wire.
Clause 33. The pressure guidewire of Clause 16, wherein the pressure wire lead is an optical fiber.
Clause 34. The pressure guidewire of Clause 16, wherein the pressure wire lead is an electrical wire.
Clause 35. The pressure guidewire of Clause 16, wherein at least a first conductive section of the connector tube and a second conductive section of the connector tube are exposed from the outer tube, the second conductive section being spaced apart from the first conductive section.
Clause 36. A pressure guidewire comprising:
   a connector tube extending from a proximal end of the pressure guidewire, the connector tube comprising a tube wall and a lumen extending therethough;
   a core wire extending distally of a distal end of the connector tube;
   a pressure sensor assembly comprising:
      a pressure sensor;
      a pressure wire lead extending from the pressure sensor toward the proximal end of the pressure guidewire; and
      a sensor housing positioned over the pressure sensor; and
   an insulator portion surrounding at least a portion of the connector tube and extending proximally of the sensor housing, the insulator portion having a proximal end and a distal end;
   wherein at least a first conductive section of the connector tube and a second conductive section of the connector tube are exposed from the insulator portion, the second conductive section being spaced apart from the first conductive section.
Clause 37. The pressure guidewire of Clause 36, wherein the pressure sensor is coaxial with the core wire.
Clause 38. The pressure guidewire of Clause 36, wherein the first conductive section is located at a proximal end of the pressure guidewire and the second conductive section is located distal of the first conductive section.
Clause 39. The pressure guidewire of Clause 38, wherein the second conductive section is positioned between the proximal end of the insulator portion and the distal end of the insulator portion.
Clause 40. A pressure guidewire comprising:
   an insulated outer tube comprising a lumen extending through the insulated outer tube;
   a pressure sensor assembly comprising a pressure sensor and a pressure wire lead extending from the pressure sensor toward a proximal end of the pressure guidewire;
   a first conductive portion within the lumen and exposed from the insulated outer tube; and
   a second conductive portion within the lumen and exposed from the insulated outer tube, the second electrically conductive portion spaced apart from the first electrically conductive portion.
Clause 41. The pressure guidewire of Clause 40, wherein the first conductive section is located at a proximal end of the pressure guidewire and the second conductive section is located distal of the first conductive section.
Clause 42. A pressure guidewire comprising:
   connector tube extending from a proximal end of the pressure guidewire, the connector tube comprising a tube wall and a lumen extending therethough;
   a core wire extending distally of a distal end of the connector tube, the core wire comprising a reduced diameter portion;
   a coil portion positioned distal to the distal end of the connector tube, the coil portion surrounding at least a portion of the core wire; and
   a pressure sensor assembly comprising a pressure sensor and a pressure wire lead extending from the pressure sensor toward the proximal end of the pressure guidewire, the pressure sensor positioned radially between the reduced diameter portion of the core wire and the coil portion such that fluid may flow through the spacing in the coil portion to the pressure sensor,
   wherein a first section of the pressure wire lead is concentric with the connector tube and a second section of the pressure wire lead is off-axis relative to a longitudinal axis of the connector tube, the second section of the pressure wire lead positioned radially outward of the core wire.
Clause 43. The pressure guidewire of Clause 42, wherein a proximal end of the coil portion is positioned radially outward of the reduced diameter portion of the core wire.
Clause 44. The pressure guidewire of Clause 42, wherein the core wire is concentric with the connector tube.
Clause 45. The pressure guidewire of Clause 42, wherein the coil portion is longitudinally displaced from the distal end of the connector tube.
Clause 46. The pressure guidewire of Clause 42, wherein the pressure wire lead is sealed to the connector tube to prevent fluid from flowing proximally to a proximal end of the pressure guidewire.
Clause 47. The pressure guidewire of Clause 42, wherein the connector tube comprises a conductive material.
Clause 48. The pressure guidewire of Clause 42, further comprising an insulator portion forming an outer surface of at least a portion of the pressure guidewire.
Clause 49. The pressure guidewire of Clause 42, further comprising an insulator portion extending longitudinally between the distal end of the connector tube and the coil portion. Clause 50. The pressure guidewire of Clause 42, wherein the tube wall of the connector tube comprises an opening configured to permit the pressure wire lead to transition from the first section that is concentric with the connector tube to the second section that is off-axis relative to the longitudinal axis of the connector tube.
Clause 51. The pressure guidewire of Clause 50, wherein the opening is sealed to prevent fluid from flowing into pressure guidewire through the opening.
Clause 52. The pressure guidewire of Clause 42, wherein at least a portion of the core wire is disposed in the lumen of the connector tube.
Clause 53. The pressure guidewire of Clause 42, further comprising a connector extending between the connector tube and the coil portion, the connector comprising an opening configured to permit the pressure wire lead to transition from the first section that is concentric with the connector tube to the second section that is off-axis relative to the longitudinal axis of the connector tube.
Clause 54. The pressure guidewire of Clause 53, wherein the pressure guidewire comprises a second coil portion extending proximally from the connector.
Clause 55. The pressure guidewire of Clause 54, wherein the second coil portion extends along a majority of a working length of the pressure guidewire.
Clause 56. The pressure guidewire of Clause 42, wherein the pressure guidewire comprises a substantially uniform outer diameter.
Clause 57. The pressure guidewire of Clause 42, wherein the pressure wire lead is an optical fiber.
Clause 58. The pressure guidewire of Clause 42, wherein the pressure wire lead is an electrical wire.
Clause 59. The pressure guidewire of Clause 42, further comprising a sensor housing positioned over the pressure sensor, the sensor housing positioned radially between the core wire and the coil portion.
Clause 60. The pressure guidewire of Clause 59, wherein the sensor housing is glued or welded to the core wire.
Clause 61. The pressure guidewire of Clause 42, wherein the coil portion comprises a sensor housing section that is stiffer than another section of the coil portion, the pressure sensor being disposed within the sensor housing section of the coil portion.
Clause 62. The pressure guidewire of Clause 61, wherein the sensor housing section of the coil portion comprises one or more openings to allow fluid to reach the pressure sensor. Clause 63. The pressure guidewire of Clause 42, further comprising a distal tip positioned at a distal end of the pressure guidewire.
Clause 64. The pressure guidewire of Clause 63 wherein the distal tip is adhered to the distal section of the core wire.
Clause 65. The pressure guidewire of Clause 63, wherein the distal tip is welded to the coil portion.
Clause 66. The pressure guidewire of Clause 63, wherein the distal tip is an enlarged distal end of the core wire.
Clause 67. A pressure guidewire comprising:
   an outer tube comprising a lumen extending through the outer tube, the outer tube comprising a coil portion;
   a core wire positioned in the lumen of the outer tube, the core wire comprising a reduced diameter portion; and
   a pressure sensor assembly comprising a pressure sensor and a pressure wire lead extending from the pressure sensor toward a proximal end of the pressure guidewire, the pressure sensor being positioned radially between the reduced diameter portion of the core wire and the coil portion,
   wherein at least a portion of the pressure wire lead is not concentric with the outer tube.
Clause 68. The pressure guidewire of Clause 67, wherein the outer tube comprises an opening configured to permit the pressure wire lead to transition from a first section that is concentric with the outer tube to a second section comprising the portion of the pressure wire lead that is not concentric with the outer tube.
Clause 69. The pressure guidewire of Clause 68, wherein the opening is sealed to prevent fluid from flowing into the pressure guidewire through the opening.
Clause 70. The pressure guidewire of Clause 67, wherein the pressure guidewire is configured to induce rapid pacing.
Clause 71. The pressure guidewire of Clause 57, wherein a proximal end of the coil portion is positioned radially outward of the reduced diameter portion of the core wire.
Clause 72. The pressure guidewire of Clause 67, wherein the outer tube comprises an insulator portion proximal to the coil portion.
Clause 73. The pressure guidewire of Clause 67, wherein the outer tube comprises connector tube comprising a conductive material.
Clause 74. The pressure guidewire of Clause 67, wherein the pressure wire lead is sealed to the other tube to prevent fluid from flowing proximally to a proximal end of the pressure guidewire.
Clause 75. The pressure guidewire of Clause 67, wherein the outer tube comprises a proximal portion comprising conductive tube, an intermediate portion comprising an insulator portion, and a distal portion comprising the coil portion.
Clause 76. The pressure guidewire of Clause 67, wherein the outer tube comprises a substantially uniform diameter.
Clause 77. The pressure guidewire of Clause 57, wherein the core wire extends through the entire lumen of the outer tube.
Clause 78. The pressure guidewire of Clause 67, wherein the pressure sensor assembly further comprises a sensor housing positioned over the pressure sensor.
Clause 79. The pressure guidewire of Clause 67, wherein the entire outer tube is the coil portion.
Clause 80. The pressure guidewire of Clause 67, wherein the coil portion extends along a majority of a working length of the pressure guidewire.
Clause 81. The pressure guidewire of Clause 67, wherein the coil portion extends along substantially the entire working length of the pressure guidewire.
Clause 82. The pressure guidewire of Clause 67, wherein the coil portion is a flat ribbon coil.
Clause 83. The pressure guidewire of Clause 67, wherein the pressure wire lead is an optical fiber.
Clause 84. The pressure guidewire of Clause 67, wherein the pressure wire lead is an electrical wire.
**Clause** 85. The pressure guidewire of Clause 67, further comprising a distal tip positioned at a distal end of the pressure wire.
Clause 86. The pressure guidewire of Clause 85, wherein the distal tip is adhered to the distal section of the core wire.
Clause 87. The pressure guidewire of Clause 85, wherein the distal tip is welded to the coil portion.
Clause 88. The pressure guidewire of Clause 85, wherein the distal tip is an enlarged distal end of the core wire.
Clause 89. A method of deploying a replacement heart valve, comprising:
   accessing a blood flow passage of a patient at an access point;
   advancing a pressure guidewire through the access point to a location adjacent to a heart valve of the patient, the heart valve to be replaced;
   advancing a pressure sensing device separate from the pressure guidewire to adjacent to the heart valve of the patient;
   advancing a delivery device having a replacement heart valve coupled therewith over the pressure guidewire;
   sensing pressure using the pressure sensing device on a first side of the heart valve to be replaced;
   sensing pressure using the pressure guidewire on a second side of the heart valve to be replaced; and
   deploying the replacement heart valve over the heart valve to be replaced.
Clause 90. The method of Clause 89, wherein the heart valve to be replaced is an aortic valve.
Clause 91. The method of Clause 89, wherein the first side is in the aorta and the second side is in the left ventricle.
Clause 92. The method of Clause 89, wherein the first side is in the left ventricle and the second side is in the aorta.
Clause 93. The method of Clause 89, wherein the heart valve to be replaced is a mitral valve.
Clause 94. The method of Clause 89, wherein the first side is in the left atrium and the second side is in the left ventricle.
Clause 95. The method of Clause 89, wherein the first side is in the left ventricle and the second side is in the left atrium.
Clause 96. The method of Clause 89, wherein the pressure sensing device is a pigtail catheter.
Clause 97. The method of Clause 89, further comprising calculating a pressure gradient across the replacement valve.
Clause 98. The method of Clause 89, further comprising calculating valve regurgitation of the replacement valve.
Clause 99. The method of Clause 89, further comprising equalizing pressure measurements between the pressure sensing device and the pressure guidewire.
Clause 100. The method of Clause 99, wherein equalizing pressure measurements occurs in the ventricle.
Clause 101. The method of Clause 99, wherein equalizing pressure measurements occurs in the aorta.
Clause 102. The method of Clause 99, wherein equalizing pressure measurements comprises automatically adjusting a phase delay between the pressure curves generated from the pressure sensing device and the pressure guidewire.
Clause 103. The method of Clause 99, wherein equalizing pressure measurements comprises manually adjusting a phase delay between the pressure curves generated from the pressure sensing device and the pressure guidewire.
Clause 104. The method of Clause 89, further comprising inducing rapid pacing through the pressure guidewire.
Clause 105. A method of assessing and/or treating a cardiac condition, comprising:
   advancing an access catheter through the vasculature of a patient;
   advancing a delivery system to the patient's heart through the access catheter;
   advancing a pressure sensing catheter to a first position; and
   advancing a pressure guidewire through the access catheter to a second position different from the first position.
Clause 106. The method of Clause 105, wherein the delivery system delivers a replacement valve to the patient's heart.
Clause 107. The method of Clause 105, wherein the delivery system delivers a dilation balloon to the patient's heart.
Clause 108. The method of Clause 105, wherein the first position is in the aorta and the second position is in the left ventricle.
Clause 109. The method of Clause 105, wherein the first position is in the left atrium and the second position is in the left ventricle.
Clause 110. The method of Clause 105, wherein the pressure sensing catheter is a pigtail catheter.
Clause 111. The method of Clause 105, wherein the delivery system comprises the pressure sensing catheter.
Clause 112. The method of Clause 105, further comprising inducing rapid pacing using the pressure guidewire.
Clause 113. The method of Clause 105, further comprising equalizing pressure measurements between the pressure sensing catheter and the pressure guidewire.
Clause 114. The method of Clause 113, wherein equalizing pressure measurements occurs in the ventricle.
Clause 115. The method of Clause 113, wherein equalizing pressure measurements occurs in the aorta.
Clause 116. The method of Clause 113, wherein equalizing pressure measurements comprises automatically adjusting a phase delay between the pressure curves generated from the pressure sensing device and the pressure guidewire.
Clause 117. The method of Clause 113, wherein equalizing pressure measurements comprises manual adjusting a phase delay between the pressure curves generated from the pressure sensing device and the pressure guidewire.
Clause 118. A method of treating a structural heart condition, comprising:
   accessing a blood flow passage of a patient at an access point;
   advancing an access catheter through the access point to a location in the heart;
   advancing a pressure guidewire through the access catheter;
   sensing pressure using the pressure guidewire; and
   inducing rapid pacing through the pressure guidewire.
Clause 119. The method of Clause 118, further comprising delivering a replacement valve to the heart through the access catheter.
Clause 120. The method of Clause 118, further comprising advancing a pressure sensing device; and sensing pressure using the pressure sensing device.
Clause 121. The method of Clause 118, further comprising connecting a current generator to the pressure guidewire.
Clause 122. The method of Clause 121, wherein connecting the current generator comprises connecting the current generator to a proximal end of the pressure guidewire.
Clause 123. The method of Clause 121, wherein connecting the current generator comprises connecting the current generator to the pressure guidewire at a location spaced distally from a proximal end of the pressure guidewire.
Nonetheless, for the avoidance of doubt, please note that the scope of the invention is to be defined by the appended claims.

## Claims

1. A pressure guidewire comprising:
an outer tube comprising a sensor housing and a coil portion;
a connector tube positioned radially inward of the outer tube, the connector tube comprising a tube wall and a lumen extending therethrough;
a core wire positioned radially inward of the outer tube and longitudinally spaced apart from a distal end of the connector tube, the core wire comprising a reduced diameter portion;
a pressure sensor assembly comprising a pressure sensor and a pressure wire lead extending from the pressure sensor toward a proximal end of the pressure guidewire, the pressure sensor being distal of the distal end of the connector tube, the pressure wire lead at least partially extending through the lumen of the connector tube; and
a distal tip at a distal end of the outer tube.

2. The pressure guidewire of claim 1, wherein the outer tube comprises a uniform diameter.

3. The pressure guidewire of claim 1, wherein the pressure wire lead is sealed to the connector tube to prevent fluid from flowing proximally to a proximal end of the guidewire.

4. The pressure guidewire of claim 1, wherein the pressure sensor is positioned radially between the outer tube and the core wire.

5. The pressure guidewire of claim 1, wherein the connector tube comprises a conductive material.

6. The pressure guidewire of claim 1, wherein at least a portion of the pressure wire lead is offset from a longitudinal axis of the connector tube, and optionally,
wherein the pressure wire lead is configured to transition from a first section that is concentric with the connector tube and a second section comprising the portion of the pressure wire lead that is offset from the longitudinal axis of the connector tube.

7. The pressure guidewire of claim 1, wherein the pressure sensor is positioned within the coil portion.

8. The pressure guidewire of claim 1, wherein the outer tube further comprises a sensor housing having at least one opening.

9. The pressure guidewire of claim 8, wherein one or more of:
the at least one opening allows fluid to flow in the space between the connector tube and the core wire;
the pressure sensor is positioned within the sensor housing; and
the pressure guidewire comprises a second coil portion extending proximally from the sensor housing, and optionally, wherein the second coil portion extends from the sensor housing toward a proximal end of the pressure guidewire.

10. The pressure guidewire of claim 1, wherein the outer tube further comprises an insulator portion extending proximally of the coil portion.

11. The pressure guidewire of claim 1, wherein the distal tip is adhered to the distal section of the core wire.

12. The pressure guidewire of claim 1, wherein the distal tip is welded to the coil portion.

13. The pressure guidewire of claim 1, wherein the distal tip is an enlarged distal end of the core wire.

14. The pressure guidewire of claim 1, wherein the pressure wire lead is an optical fiber or an electrical wire.

15. The pressure guidewire of claim 1, wherein at least a first conductive section of the connector tube and a second conductive section of the connector tube are exposed from the outer tube, the second conductive section being spaced apart from the first conductive section.
